# EUROPEAN PATENT SPECIFICATION

(11) **EP 3 755 687 B1**
(45) Date of publication and mention of the grant of the patent: **29.06.2022**
(21) Application number: 19721010.7
(22) Date of filing: 21.02.2019
(51) Int. Cl.: C07D 207/323, C07D 207/325, C07F 7/18, C01B 32/20

(54) **ADDUCT BETWEEN A PYRROLIC COMPOUND AND AN INORGANIC OXIDE HYDROXIDE, A SUPER-ADDUCT BETWEEN A PYRROLIC COMPOUND, AN INORGANIC OXIDE HYDROXIDE AND A CARBON ALLOTROPE, ELASTOMERIC COMPOSITION COMPRISING THE SUPER-ADDUCT AND METHODS FOR PRODUCING THE SAME**
ADDUKT ZWISCHEN EINER PYRROLVERBINDUNG UND EINEM ANORGANISCHEN OXIDHYDROXID, SUPERADDUKT ZWISCHEN EINER PYRROLVERBINDUNG, EINEM ANORGANISCHEN OXIDHYDROXID UND EINEM KOHLENSTOFFALLOTROP, ELASTOMERE ZUSAMMENSETZUNG MIT DEM SUPERADDUKT UND VERFAHREN ZUR HERSTELLUNG DAVON
PRODUIT D'ADDITION ENTRE UN COMPOSÉ PYRROLIQUE ET UN HYDROXYDE D'OXYDE INORGANIQUE, UN SUPER-PRODUIT D'ADDITION ENTRE UN COMPOSÉ PYRROLIQUE, UN HYDROXYDE D'OXYDE INORGANIQUE ET UN ALLOTROPE DE CARBONE, COMPOSITION ÉLASTOMÈRE COMPRENANT LE SUPER-PRODUIT D'ADDITION ET LEURS PROCÉDÉS DE PRODUCTION

(30) Priority: 21.02.2018 IT 201800002919; 09.05.2018 IT 201800005206
(43) Date of publication of application: 30.12.2020
(73) Proprietor: Pirelli Tyre S.p.A., 20126 Milano (IT)
(72) Inventor: GALIMBERTI, Maurizio Stefano, 20133 Milano (IT); BERNARDI, Andrea, 20133 Milano (IT); BARBERA, Vincenzina, 20133 Milano (IT); LOCATELLI, Daniele, 20133 Milano (IT)
(74) Representative: Susanetto, Carlo
(86) International application number: PCT/IB2019/051422
(87) International publication number: WO 2019/162873

(56) References cited:
- WO-A1-2016/050887
- WO-A1-2018/087688
- Minna Poikelispää: "Improvements of Nanofiller-Elastomer Systems by Filler Modification and Tailored Mixing Techniques", Doctoral dissertation, 12 October 2017 (2017-10-12), pages 1459-2045, XP055507520, Retrieved from the Internet: URL:https://tutcris.tut.fi/portal/files/12 969277/poikelisp_1492.pdf [retrieved on 2018-09-17]

## Description

The present invention relates to an adduct formed of a pyrrolic compound and an inorganic oxide hydroxide, to a super-adduct formed of said adduct and a carbon allotrope, to an elastomeric composition comprising the super-adduct and to a few methods for producing the same, which methods comprise the features stated in the preamble of the independent claims.

It is known that some materials are distributed and dispersed in liquid or solid matrices in order to boost specific and desired characteristics thereof.

The objects of carrying out the mixing operation are: obtaining a complete and homogeneous distribution of the material in the matrix and dispersing said material, i.e. reducing the dimensions of the agglomerates of the material to particles that cannot be divided any further by means of thermomechanical stress. These particles can be the fundamental units that constitute the material, or are aggregates of these units. Scientific and technological research has always invested a lot of effort into achieving optimum distribution and dispersion. These efforts have increased in the last few years, following the appearance of materials having nanometric dimensions. A material having nanometric dimensions has a high surface area, and is therefore more difficult to distribute and disperse in matrices having a different chemical nature. It is known that two main factors influence the distribution and dispersion of a material in a matrix, whether it be a liquid or solid matrix: the chemical nature of materials and matrices and the mechanical energy used to mix the substances. It is known that mixing provides better distribution and dispersion the more similar the chemical nature of the substances is and the greater the mechanical stress is that is applied. Various mixing techniques are well known, both for solid materials and for liquid mixtures. One parameter used in chemistry to provide chemical compatibility between one substance and other substances is the solubility parameter. The term "solubility parameter" can be explained by recalling the medieval rule of thumb *similia similibus solvuntur,* according to which two substances form a solution provided that they have a similar chemical nature. Two solubility parameters are known: the Hildebrand solubility parameter and the Hansen solubility parameter. The Hildebrand solubility parameter is defined as the square root of the cohesive energy density, wherein the cohesive energy density is the amount of energy required to completely remove a molecule (unit volume) from a collection of (identical) molecules and carry it for an infinite distance. The Hansen solubility parameters are based on the concept that a substance is similar to another substance if between them the molecules that it comprises stabilise interactions that are similar to those that stabilise with the molecules of the other substance. Three Hansen solubility parameters are defined: one relates to interactions caused by dispersion forces, one relates to interactions that involve dipoles, and one results from the interactions caused by hydrogen bonds. The three parameters are treated as coordinates of a point in 3 dimensions known as the Hansen space. The closer two molecules are in the Hansen space, the more likely they are to form a solution. The so-called Hansen sphere gives this expression a quantitative dimension. The sphere is derived from the data relating to the solubility of a substance and is defined by a radius, known as the interaction radius. If the coordinates of another substance fall within the sphere, it can be soluble. All of these assessments relate to the enthalpy aspects of a mixture. In order to be soluble, the value for the free mixing energy needs to be negative and therefore the entropic aspects are also evaluated. However, having similar solubility parameter values is a necessary condition in order to at least have compatibility between two substances, if not solubility, and, going back to that written above, optimum distribution and dispersion of one substance in another. In the industrial sector, one of the significant application examples with respect to the distribution and dispersion of a material in a matrix relates to the distribution and dispersion of sp²-hybridized carbon allotropes in liquids or in polymer matrices.

Carbon is a chemical element having an atomic number Z = 6, which can exist in several allotropic forms. In chemistry, allotropes are formed of chemical elements that differ in the way in which the atoms are bonded to one another, and that therefore have different chemical and physical properties. Carbon allotropes can be classified according to the hybridization of the carbon atoms. Carbon allotropes exist in which the carbon is sp³-hybridised and carbon allotropes exist in which the carbon is sp²-hydrbidised. In diamonds, the carbon atoms are sp³-hybridised. The carbon atoms are sp²-hydbridised in carbon allotropes such as carbon black, graphites, nanographites, graphene, fullerene and carbon nanotubes. There are a lot of carbon allotropes in which the carbon is sp²-hydridised. These include: nanotoroids, nanohorns, graphene nanobelts, and several others. The article by M. Terrones et al. [NANO TODAY 5.4 (2010) 351-372] describes the number of sp²-hybridised allotropes. Furthermore, the article Carbon, 2016, 98, 708-732 outlines the ever-growing study and development of new forms of sp²-hybridised carbon allotropes. This article also states that the challenge is to provide the compatibilisation of these allotropes in "3D *architectures",* therefore in composite materials based on different types of matrices.

Graphene is a carbon allotrope in which the carbon is sp²-hydbridised. Graphene consists of a layer of carbon atoms having the thickness of a single carbon atom; it has a polymeric nature and the repeating units are aromatic cycles, typically having 5 or 6 carbon atoms. According to a widespread representation of the bonds and the distribution of the electrons in an aromatic ring, every carbon atom is sp²-hybridised and uses two of these hybrid bonds to form bonds with two other carbon atoms and the third sp²-hybridised orbital to form a bond with the hydrogen atom. The lateral superposition between the 2p orbitals brings about the formation of the π orbitals. The electrons involved in the π orbitals are therefore relocated on the polycyclic aromatic system. Graphene is therefore a polycondensed aromatic system. Examples of aromatic polycondensates are perylene, pyrene, phenanthrene and anthracene.

Stacked layers of graphene form graphite, which may have a variable number of layers up to several thousands. So-called nanographites are formed when there is a small number of stacked layers. Carbon nanotubes having a single wall or multiple walls can be considered to be formed by layers of rolled graphene. Carbon black is formed of spheres that are bonded together by covalent bonds and comprise less than ten packets consisting of a few graphene layers arranged in a disorderly fashion.

Carbon allotropes having sp²-hybridised carbon have different degrees of flatness. A system is defined as being aromatic when it satisfies three conditions: (a) the system is cyclic, (b) the atoms involved in the cycle are all sp²-hybridised and the sum of the electrons π satisfies Hückel's rule (π + 4n+2, where n is an integer including zero), (c) the system is planar. In the case of graphene, all of the requirements are met. Carbon allotropes that are not planar, such as fullerene and carbon nanotubes, may, however, be defined as being aromatic despite the curvature influencing the condition of flatness.

Sp²-hybridised carbon allotropes are traditionally divided into "nano" and "nano-structured". An individual chemical is defined as being "nano" when at least one dimension thereof is smaller than 100 nm. Carbon allotropes such as fullerene, graphene and nanographites and carbon nanotubes, are "nano" allotropes. In fact, fullerene has dimensions smaller than 100 nm. Carbon nanotubes have a diameter of a few nanometres. As stated above, a nanographite is formed of crystalline aggregates formed by a few stacked layers of graphene. Therefore, the dimension of the crystalline aggregate in the direction that is orthogonal to the layers ranges from a few nm to a few tens of nanometres. Graphene has the thickness of one carbon atom and therefore has a nanometric dimension. Carbon black, which has been used for over a century for reinforcing polymer materials and for several other applications, is conversely "nanostructured". In fact, it comprises fundamental spherical particles, which have nanometric dimensions and are connected so as to form aggregates in which said fundamental particles are held together by covalent bonds. Said aggregates have dimensions of more than 100 nm.

As a result of that described, specifically the aromatic structure and the macrostructure, sp²-hybridised carbon allotropes are very electrically and thermally conductive and have key mechanical properties. Taking into account cost-benefit ratios, too, which are significantly optimised by carbon black and graphite and by means of the nanofillers during the optimisation stage, above all the carbon nanotubes, sp²-hybridised carbon allotropes, both nano-carbon and nanostructured, have several diverse, important large-volume applications.

For example, carbon black has been used since the beginning of the 20^{th} century for dynamic-mechanical reinforcement of elastomeric compounds. The elastomeric compounds have large-volume applications in tyres. The compounds for tyres are largely reinforced by carbon black. As already mentioned, carbon black is nanostructured. "Structure" defines the organisation created by the spheres that aggregate, leaving empty spaces therebetween. The more empty spaces there are between the spheres, the greater the structure. During the mixing process for preparing an elastomeric compound, for example for tyres, the elastomer is able to enter the gaps in the carbon black, to immobilise and transform into a rigid phase. This mechanism considerably contributes to the reinforcement of fused elastomers and polymers under high deformation. As mentioned above, the object of the mixing process is to optimally distribute and disperse the materials that are dispersed in a matrix, in this case the carbon black in the polymer matrix. However efficient the mixing process is, it is very difficult to fully separate the carbon black aggregates. Even if this result were achieved, during ageing and application of the composite material, the carbon black aggregates come into contact with one another for obvious thermodynamic reasons, by means of Van der Waals forces. Agglomerates are therefore created that may, however, be separated into the starting aggregates by means of thermomechanical stresses. This phenomenon is described in the article "Polymer 51" (2010) 2057-2068 by Bohm et al.

This article illustrates that, although optimum mixing is achieved, agglomerates form as a result of storage of the compound. This therefore results in a phenomenon whereby the agglomerates are formed and destroyed.

This phenomenon is known as *filler networking* and is typically researched by carrying out dynamic mechanical tests in the presence of small deformations, typically of up to 10 % and no more than 25 %. By means of these tests, the storage modulus, the loss modulus and the complex modulus of the elastomeric composite material are determined. If the *filler networking* phenomenon is present, it is noted that the complex modulus and the storage modulus decrease as the extent of the deformation increases from the minimum value to the values indicated above. This reduction in the modulus clearly indicates that the modulus does not only depend on the time when the stress was applied, but also on the extent of the deformation, and therefore indicates the non-linearity of the modulus. The reduction in the modulus or the non-linearity of the modulus is known as the Payne effect, named after the scientist who was first to rationalise it. In the same deformation range, the loss modulus passes a maximum. In fact, the *filler networking* phenomenon is typically a dissipative phenomenon. The relationship between the loss modulus and the storage modulus, known as the tangent of δ (tan δ), is indicative of the dissipation of energy for a given value for the storage modulus. In one dynamic mechanical application of an elastomeric composite material, tan δ indicates the hysteresis generated by the material. Dissipation of energy and hysteresis typically imply the generation of heat. Therefore, the Payne effect occurs when the *filler networking* phenomenon is present, specifically when filler agglomerates are present. In order for these agglomerates to be present, the filler has to reach percolation, i.e. has to construct a continuous path inside the elastomeric composite material. However, it is also possible for the modulus to reduce after the filler of the polymer matrix has been removed, independently of the quantity of filler present in the composite material. Both these interpretations explain the Payne effect. The first interpretation (breaking down the agglomerates) is the dominant one. Of course, the phenomenon of breaking down the agglomerates is the dominant one in elastomeric compounds for applications such as in compounds for tyres. It is clear that better dispersion of the filler and better compatibilisation thereof with the polymer matrix lead to a lesser degree of *filler networking* and a smaller Payne effect.

In the case of compounds for tyres, *"filler networking"* contributes to the road-holding performance and to the energy consumption and therefore to the tyres' impact on the environment.

It would be extremely desirable for the applications of composite materials consisting of elastomers and carbon black in compounds for tyres to be able to achieve the highest amount of reinforcement together with low energy dissipation. Therefore, it would be extremely desirable to achieve optimum dispersion of the carbon black in the elastomeric matrix. Optimum dispersion of the carbon black in an elastomeric matrix is therefore an aspect of primary importance with respect to the properties of the composite material and is actually constantly the focus of research efforts, both in the academic and the industrial field.

There are already a wide variety of applications for nanometric carbon allotropes such as carbon nanotubes, nanographites and graphenes, which are recalled in the introduction of the article: S. Musto, V. Barbera, V. Cipolletti, A. Citterio, M. Galimberti, "Master curves for the sulphur assisted crosslinking reaction of natural rubber in the presence of nano- and nano-structured sp2 carbon allotropes" EXPRESS POLYMER LETTERS vol.11, no.6 (2017) 435-448 and in chapter 8 "Controlled Functionalization of Graphene Layers", of the book "GRAPHENE MATERIALS - STRUCTURE, PROPERTIES AND MODIFICATIONS," by M. Galimberti, V. Barbera, A. Sironi, InTech, ISBN 978-953-51-3140-3, for example.

In particular, carbon nanotubes have exceptional electrical and mechanical properties. As a result of the nature of a nanofibre, said nanotubes have a high surface area and therefore a high interfacial area. This causes them to easily form continuous lattices and therefore to construct paths for electrical and thermal conductivity. In addition, a high interfacial area with the matrix makes them capable of transmitting their properties to the composite material very effectively. The high surface area is a typical consequence of a nanometric dimension that characterises the nanofillers, such as carbon nanotubes and nanographites and graphene. The following articles demonstrate the importance of the high surface area and therefore high interfacial area in a material comprising sp²-hybridised carbon allotropes: Galimberti M., Coombs M., Riccio P., Ricco' T., Passera S., Pandini S., Conzatti L., Ravasio A., Tritto I., "The Role of CNTs in Promoting Hybrid Filler Networking and Synergism with Carbon Black in the Mechanical Behavior of Filled Polyisoprene", MACROMOL. MATER. ENG., 298 (2012), 241-251 and S. Agnelli, V. Cipolletti, S. Musto, M. Coombs, L. Conzatti, S. Pandini, T. Riccò, M. Galimberti, "Interactive effects between carbon allotrope fillers on the mechanical reinforcement of polyisoprene based nanocomposites", EXPRESS POLYMER LETTERS 8(6) (2014) 436.

In the case in which the nanometric fillers, therefore fillers having a high surface area, are used in fused elastomers and polymers, said nanometric fillers percolate, that is they create a continuous path inside the polymer material, the so- called filler "network", which has a low nanometric filler content. Therefore, the composite material is made electrically conductive by the use of a small amount of the nanometric filler. This result is particularly important for compounds that contain fillers, such as silica, which do not conduct the electrical current. It is sufficient to use a small amount of carbon nanofiller in order to provide electrical conductivity. This result is very important. One need only consider the compounds based on silica that are used in tyre treads. These suffer from an accumulation of electrostatic energy if their electrical conductivity is not provided, at least on an antistatic level. Obtaining optimum distribution and dispersion of the sp²-hybridised carbon allotropes in an elastomeric matrix is therefore an object of great importance. However, the formation of the so-called nanometric filler "network", a "network" based on Van der Waals forces, leads to the filler networking phenomenon, namely a continuous undoing and reforming of the "network". This causes a large amount of energy dissipation that, as already stated, is responsible for the road-holding ability of a tyre, for example, but also for the energy consumption and therefore the impact on the environment. Therefore, not only the optimum dispersion of nanometric fillers in an elastomeric matrix but also the compatibilisation of the fillers with the polymer matrix are constantly the object of research efforts in both the academic and industrial field.

As mentioned above, it is possible to use mechanical energy to optimally distribute and disperse a filler in a polymer matrix.

However, mechanical mixing of polymer composite materials that contain carbon nanotubes causes said nanotubes to fracture, as shown in Galimberti M., Coombs M., Riccio P., Ricco' T., Passera S., Pandini S., Conzatti L., Ravasio A., Tritto I., "The Role of CNTs in Promoting Hybrid Filler Networking and Synergism with Carbon Black in the Mechanical Behavior of Filled Polyisoprene", MACROMOL. MATER. ENG., 298 (2012), 241-251.

Nanometric and nano-structured carbon allotropes can be mixed together and used in composite materials so as to form hybrid systems, as shown in the articles cited above, Galimberti M., Coombs M., Riccio P., Ricco' T., Passera S., Pandini S., Conzatti L., Ravasio A., Tritto I., "The Role of CNTs in Promoting Hybrid Filler Networking and Synergism with Carbon Black in the Mechanical Behavior of Filled Polyisoprene", MACROMOL. MATER. ENG., 298 (2012), 241-251 and S. Agnelli, V. Cipolletti, S. Musto, M. Coombs, L. Conzatti, S. Pandini, T. Riccò, M. Galimberti, "Interactive effects between carbon allotrope fillers on the mechanical reinforcement of polyisoprene based nanocomposites", EXPRESS POLYMER LETTERS 8(6) (2014) 436.

One book chapter that discloses composite materials having hybrid filler systems is M. Galimberti, S. Agnelli, V. Cipolletti, "Hybrid filler systems in rubber nanocomposites", chapter 11 of "PROGRESS IN RUBBER NANOCOMPOSITES 1STEDITION" Hardcover ISBN: 9780081004098, eBook ISBN: 9780081004289, Editors: Sabu Thomas Hanna Maria Imprint: Woodhead Publishing, Published Date: 10th November 2016, Page Count: 596. As shown in these documents, too, the application of mechanical energy also causes the carbon nanotubes to fracture in the presence of a hybrid system.

In order to facilitate the distribution and dispersion of sp²-hybridised carbon allotropes having nanometric dimensions in polymer matrices, it may be useful to prepare pre-dispersed allotropes, in which the concentration of the allotropes is typically greater than the concentration of those used in the composite material and the finished product. However, the application of mechanical energy damages the structure of the nanometric fillers, for example fractures the carbon nanotubes. Furthermore, the dispersion achieved by means of the mechanical mixing process does, however, not achieve the object of preventing the phenomenon of the agglomeration of allotrope particles and aggregates - proven, for example, by the fact that all the composite materials in the elastomeric matrix are characterised by hysteresis caused by the filler networking phenomenon. Therefore, the preparation of pre-dispersed allotropes does not render the carbon allotrope more compatible with the polymer matrix.

In order to effectively disperse the carbon nanotubes in the polymer matrix without considerably damaging the structure of said nanotubes, it is necessary to have recourse to using solvents to aid with mixing, typically organic solvents such as toluene, solvents that are not desirable in industrial development due to their environmental problems.

Sp²-hybridised carbon allotropes therefore have an important industrial presence, a large variety of sp²-hybridised carbon allotropes are already available and several others are reasonably close to development and availability. In the light of the above, it seems to be increasingly important and relevant to achieve optimum distribution and dispersion of the sp²-hybridised carbon allotropes in liquid or solid matrices.

In fact, various studies and tests carried out in the lab have shown that the typical thermomechanical stresses of the action of mixing the carbon black with the polymer matrices and the thermomechanical stresses to which the composite materials are subjected during application thereof, for example, are not capable of separating the aggregates into the fundamental components, therefore effectively transferring the specific characteristics of the dispersed species to the final composition.

The difficulty in obtaining optimum dispersion of sp²-hybridised carbon allotropes in polymer matrices considerably increases when the polymer matrix has a polar nature. In the publication by Hilmar Koerner, Weidong Liu, Max Alexander, Peter Mirau, Heather Dowty, Richard A. Vaia, POLYMER (2005), 46, 4405-4420, in order to obtain effective dispersion of a carbon nanotube in a polyurethane matrix, a solvent such as tetrahydrofuran (THF) is used.

In the case of dispersions in liquid matrices, such as those used in the world of paints and inks, it would be immensely desirable to be able to use eco-compatible solvents that are typically polar, such as alcohols, ketones, ethers and esters, and it would be even more immensely desirable to be able to use water. The difficulty of having stable carbon allotrope dispersions in liquid polar means, and particularly in water, is clear. In order to improve the dispersibility of nanotubes and, more generally, carbon nanofillers in a liquid means, in particular if the liquid means has a different chemical nature to that of the carbon allotrope, mechanical energy is used, typically by means of the use of ultrasounds. However, it is known that the use of ultrasounds leads to fracturing of the carbon nanofillers, such as carbon nanotubes and thin graphene sheets.

Therefore, a fundamental technical disadvantage of the use of sp²-hybridised carbon allotropes in both liquid polymer matrices and polymer matrices is the difficulty in obtaining optimum dispersion, in particular when only mechanical energy is used.

In fact, when the allotropes are not well dispersed in the matrix, their chemical, physical and mechanical properties are not efficiently transferred to the composite material.

In order to optimally distribute and disperse the allotropes by making use of the chemical properties of the species involved, the carbon allotrope and whatever surrounds it, whether that be a polymer matrix or a liquid means, have as similar solubility parameters as possible.

The concept behind the "solubility parameter" has already been applied to nanometric carbon allotropes, such as carbon nanotubes, in the article by Bergin, Shane D., et al. "Multicomponent solubility parameters for single-walled carbon nanotube- solvent mixtures" ACS NANO, 3(8): 2340-2350 (2009), carbon fibres, in the article by Launay, H., Charles M. H., and Kristoffer A. "Hansen solubility parameters for a carbon fiber/epoxy composite" CARBON 45(15): 2859-2865 (2007) and grapheme, in the article by Yenny Hernandez, Mustafa Lotya, David Rickard, Shane D. Bergin, and Jonathan N. Coleman, "Measurement of Multi component Solubility Parameters for Graphene Facilitates Solvent Discovery", LANGMUIR 2010 26 (5), 3208-3213. The article by Bergin et al. states the dispersibility of single-walled carbon nanotubes in different kinds of solvents. These solvents are: in N-methyl-2-pyrrolidone, 1,3-dimethyl-2-imidazolidinone, N-vinylpyrrolidone, N-dimethylacetamide, N-dimethylformamide, 1-cyclohexylpyrrolidone, N-butyl-2-pyrrolidone, N-ethyl-2- pyrrolidone, 1-benzyl-2- pyrrolidone, dimethyltetrahydro-2-pyrimidinone, oxo-pyrrolidinepropionitrile, N-octylpyrrolidone, N-dodecyl pyrrolidone, and N-formylpiperidine. The dispersibility of the nanotubes is measured by determining their concentration in dispersion after centrifugation. The article states the dependency of the dispersibility of the nanotubes on the solubility parameter (the authors state a greater dependency on the solubility parameter deduced from surface energy values rather than from cohesive energy values) of the means in which they are located. The points that describe this dependency remain around a straight line that shows a non-negligible incline. This means that even small variations in the solubility parameter of the matrix can lead to noticeable differences in the dispersibility of the nanotubes. The article also mentions a few exceptions, suggesting the need for additional in-depth analyses. In essence, the article by Bergin et al. shows that a solvent capable of minimising the free mixing energy is able to encourage *debundling* of the tubes and the exfoliation of the graphene aggregates. Furthermore, by the carbon nanotubes and the polymer matrix having very similar solubility parameters provides easier percolation of the nanotubes and greater electrical conductivity, as shown in the article Ata, S., Mizuno, T., Nishizawa, A., Subramaniam, C., Futaba, D. N., & Hata, K. (2014) "Influence of matching solubility parameter of polymer matrix and CNT on electrical conductivity of CNT/rubber composite" SCIENTIFIC REPORTS, 4:7232. In this article, nine different polymer matrices are used. These are: poly(styrene-co-butadiene) (known in the world of elastomers as SBR), poly(1,4-cis-butadiene), poly(1,4-cis-isoprene), poly(butadiene-co-acrylonitrile) (known in the world of elastomers as NBR), poly(ethylene-co-1-butene-co-acrylonitrile) (specifically hydrogenated poly(butadiene-co-acrylonitrile) (known in the world of elastomers as hydrogenated nitrile rubber), poly(ethylene oxide-co-epichlorohydrin -allyl glycidyl ether) poly(ethylene-monomer acrylic) (known in the world of elastomers as acrylic elastomers or ACM), poly(vinylidene fluoro-co-tetrafluoroethylene-co-hexafluoropropylene) (known in the world of elastomers as fluoro rubber). In practice, it has been demonstrated using experiments that the agreement between the solubility parameter of the matrix and that of the nanotubes is important for enabling the formation of a continuous lattice of nanotubes in said matrix and therefore for obtaining electrical conductivity. In fact, curves are shown that correlate the electrical conductivity with the solubility parameter of the matrix. It is stated that the most effective dispersion is obtained using fluoro rubber as the matrix. It is important to note how the composite materials that contain the nanotubes display different electrical conductivity values despite the presence of very similar values for the solubility parameters of the matrix.

Therefore, the work in the literature demonstrates that, in order to achieve effective dispersibility of carbon nanotubes in a means or a matrix, it is important to have solubility parameters that are similar for the nanotubes and for the means or the matrix.

In order to obtain better dispersion of carbon nanotubes in a polymer matrix, regardless of the solubility parameter of said matrix, it is possible to add a compatibiliser to the nanotubes. The article SCIENTIFIC REPORTS (2014), 4:7232, also states that the addition of small quantities of fluoro rubber as the polymer compatibiliser for the nanotubes leads to the most effective dispersion of said nanotubes and to greater electrical conductivity.

Another method used for improving the dispersibility of carbon allotropes in liquid or polymer matrices involves oxidation of the allotropes.

For example, methods for oxidising the carbon black and, more generally, graphite substances are known. For example, Staudenmaier's method, which is mentioned in Staudenmaier L. (1898) "Verfahren zur Darstellung der Graphitsäure" BER. DTSCH. CHEM. GES. 31: 1481-1487, which uses a mixture of H₂SO₄ and HNO₃ (2/1) with KClO₃ as the oxidant, and Hummers' method, which is mentioned in Hummers W. S., Offeman R. E. (1958) "Preparation of graphitic oxide" J. AM. CHEM. SOC. 80: 1339-1339, which uses H₂SO₄/NaNO₃ and KMnO₄.

However, Staudenmaier's method produces ClO₂ which is explosive, whereas Hummers' method forms NO₂/N₂O₄ and manganese ions, which can be difficult to remove, using strong acids that remain absorbed on the graphite substrate. Furthermore, only partial oxidation takes place.

Methods are available in scientific literature that seek to improve Hummers' method, as mentioned in the introduction to the chapter "Controlled Functionalization of Graphene Layers" in "GRAPHENE MATERIALS - STRUCTURE, PROPERTIES AND MODIFICATIONS," by M. Galimberti, V. Barbera, A. Sironi, InTech, ISBN 978-953-51-3140-3. These are all chemical reactions having atom efficiency that is lower than 100 %. Therefore, the objects of obtaining a simple and viable method that is applicable on a large scale have not been achieved. Furthermore, only oxygenated groups are introduced by means of the above-mentioned reactions.

The oxidation of carbon black was also carried out with ozone and with hydrogen peroxide. Carbon black was treated with ozone, as stated in Wang W., Vidal A., Donnet J.B., Wang M.J., KGK W., KAUTSCHUK GUMMI KUNSTSTOFFE, 46, 933, 1993, and in F. Cataldo, JOURNAL OF NANOSCIENCE AND NANOTECHNOLOGY,7,1446 (2007).

However, oxidation with ozone has to be carried out in dedicated plants and is not easily industrially scalable due to the criticality of the ozone and because it remains absorbed in the carbon black that acquires an undesirable oxidizing ability, especially if the carbon black is introduced into a polymer matrix.

Oxidation with hydrogen peroxide has been described in US 6,120,594, in which the carbon black is treated using traditional industrial equipment. This document states that, compared with treatment with ozone, lower pHs and a greater quantity of oxygen are obtained. It is clear that this procedure requires an industrial plant in order to be carried out and only leads to the introduction of oxygenated groups. It is true that the introduction of oxygenated groups can facilitate the dispersion of sp²-hybridised carbon allotropes in rubber lattices and in polar rubbers. However, it is not possible to hypothesize that oxidation can lead to better dispersion in matrices that do not have polarity characteristics.

Another method used to modify the chemical nature of sp²-hybridised carbon allotropes is the functionalisation of the sp²-hybridised carbon allotropes by means of chemical reactions that are not oxidation.

One method for functionalising the carbon black is the so-called *"cofuming technology",* which forms "*furnace carbon black"* in the presence of SiCl₄. So-called *"dual phase"* materials are obtained, which are substantially based on carbon black but also contain SiOR groups, as stated in M. J. Wang, Y. Kutsovsky, P. Zhang, L. J. Murphy, S. Laube K. Mahmud, RUBBER CHEMISTRY & TECHNOLOGY, 75, 247 (2002) and in M. J. Wang,, K. Mahmud, L. J Murphy, & W. J. Patterson, KAUTSCHUK GUMMI KUNSTSTOFFE, 51, 348 (1998). This type of carbon black has essentially been proposed in the tyre world. In fact, the carbon black becomes silanisable, that is it can react with silanes, in particular with silanes that contain ssulfurs and that encourage bonding of the filler to the elastomer. The technology that has to be realised in dedicated plants is, however, very expensive and is not applied industrially.

The carbon black was also treated with triazoles, as mentioned in J. A. Belmont, V. R. Tirumala, P. Zhang WO 2013/130099 (2013), and with polysulfides, as mentioned in W. Wampler, B. M. Jacobsson, L. Nikiel, P. D. Cameron, J. Neilsen, US 2015/0191579. However, the chemical reactions involved require reagents that can only be used within the scope of dedicated processes. Furthermore, carbon black that only has a certain type of functional group is obtained.

It therefore needs to be highlighted that all the methods mentioned above, beyond the above-mentioned specific technical problems connected with each method, require dedicated plants and are not versatile, that is only one type of functional group is introduced. As already mentioned with respect to oxidation, this may cause the functionalised carbon black to only be compatible with a certain type of matrix.

A lot of research activity is also dedicated to the functionalisation of nanographites and layers of graphene, as is analysed well in T. M.Swager, "Functional Graphene: Top-Down Chemistry of the π-Surface", ACS MACRO LETT. 1, 3 (2012) and in C.K. Chua, M. Pumera, "Covalent chemistry on graphene" CHEM. Soc. REV.42, 3222 (2013).

Oxidation is carried out in the majority of cases using the methods already described for carbon black, i.e. using chemical reactions with oxidants and strong acids. A furnace is alternatively used, in which a thermal shock is applied to an intercalated graphite sample. After this treatment, the graphite is subjected to ultrasonication and singular layers of graphene are therefore obtained. This approach is described in I. Zaman, H.C Kuan,Q. Meng, A. Michelmore, N. Kawashima, T. Pitt, L. Zhang, S. Gouda, L.Luong, J. Ma, "A Facile Approach to Chemically Modified Graphene and its Polymer Nanocomposites" ADV. FUNCT. MATER. 22, 2735 (2012). In this method, the use of a furnace and therefore ultrasonics are required. It is therefore obvious how this approach firstly requires the intercalation of the graphite, followed by the use of dedicated equipment. Furthermore, this method causes the pervasive introduction of oxygenated groups together with the profound change in the graphite structure.

Nowadays, research focuses on the functionalisation of the edges of the graphene layers in order to preserve the ideal structure of the graphene layer. Documentation is given in K. Liu, S. Chen, Y. Luo, D. Jia, H. Gao, G. Hu, L. Liu "Edge-functionalized graphene as reinforcement of epoxy-based conductive composite for electrical interconnects" COMPOSITES SCIENCE AND TECHNOLOGY 88 (2013) 84-91, in D. Bhattacharjya, I.Y. Jeon, H.Y. Park, T. Panja, J. Beom Baek, J.S. Yu "Graphene Nanoplatelets with Selectively Functionalized Edges as Electrode Material for Electrochemical Energy Storage" LANGMUIR 2015, 31, 5676-5683, in A. Narita, X.Y. Wang, X. Feng, K. Müllen, "New advances in nanographene chemistry", CHEM. SOC. REV., 2015, 44, 6616, in P. Xiong, J. Zhu, L. Zhang, X. Wang, "Recent advances in graphene-based hybrid nanostructures for electrochemical energy storage" NANOSCALE HORIZ., 2016, 1, 340-374, in Punetha, V. D., Rana, S., Yoo, H. J., Chaurasia, A., McLeskey, J. T., Ramasamy, M. S., Cho, J. W. (2017) "Functionalization of carbon nanomaterials for advanced polymer nanocomposites: A comparison study between CNT and graphene" PROGRESS IN POLYMER SCIENCE, 67, 1-47.

The functionalisations lead to the introduction of halogen atoms that can be converted into cyan groups, as mentioned in S. Ito, M. Wehmeier, J. D. Brand, C. Kübel, R. Epsch, J. P. Rabe and K. Müllen, CHEM. EUR. J., 2000, 6, 4327 and in L. Chen, X. Dou, W. Pisula, X. Y. Yang, D. Q. Wu, G. Floudas, X. L. Feng and K. Müllen, CHEM. COMMUN., 2012, 48, 702, in arylthio groups, as mentioned in L. Chen, X. Dou, W. Pisula, X. Y. Yang, D. Q. Wu, G. Floudas, X. L. Feng and K. Müllen, CHEM. COMMUN., 2012, 48, 702. The halogen groups can create *coupling* reactions catalysed by metals, as mentioned in Swain, A. K., & Bahadur, D. (2013) "Facile synthesis of twisted graphene solution from graphite-KCl" RSC ADVANCEs,3(42), 19243-19246.

Furthermore, boron atoms are introduced, as mentioned in I. A. I. Mkhalid, J. H. Barnard, T. B. Marder, J. M. Murphy, J. F. Hartwig, Chem. Rev., 2009, 110, 890, the C-H bonds are directly arylated, as mentioned in K. Mochida, K. Kawasumi, Y. Segawa and K. Itami, J. AM CHEM. SOC., 2011, 133, 10716, the C=H double bonds are activated, as mentioned in K. Ozaki, K. Kawasumi, M. Shibata, H. Ito and K. Itami, NAT. COMMUN., 2015, 6, 6251, and new aromatic structures are constructed, as mentioned in K. Kawasumi, Q. Zhang, Y. Segawa, L. T. Scott and K. ITAMI, NAT. CHEM., 2013, 5, 739.

All the methods mentioned with respect to the functionalisation of the graphene layers at the edges require complicated chemical reactions. Furthermore, they lead to the introduction of specific types of functional groups. Therefore, the graphene layers can only be compatibilised with matrices that have a solubility parameter that is similar to that of the modified allotrope.

The method already mentioned, described in I. Zaman et al, ADV. FUNCT. MATER.22, 2735 (2012), could be used to introduce various types of functional groups. The functional groups introduced on the graphene layer are reacted with a long-chain surfactant. However, this is one type of surfactant. Furthermore, the technical problems already stated remain: the alteration of the graphite structure and the complexity of the procedure.

However, all the above-mentioned methods require the application of chemical reactions that have atom efficiency that is definitely below 100 % and require the use of dedicated plants. Scientific literature also mentions the introduction of functional groups on carbon nanotubes, thus achieving covalent functionalisation, see P. Singh, S. Campidelli, S. Giordani, D. Bonifazi, A. Bianco, M. Prato, "Organic functionalisation and characterisation of single-walled carbon nanotubes", CHEM. Soc. REV 38, 2214 (2009). For example, it is possible to form aylations, dipolar 1,3-cycloadditions, radical additions, Diels-Alder cycloadditions, nucleophilic additions, introduction of carboxyl groups, of nitrene and of fluorine atoms. All these reactions lead to the introduction of just one type of functional group and require reaction conditions that can be achieved in the lab but are not easily achieved on a large scale except in dedicated reactors.

The functionalisation of the carbon nanotubes is also carried out without forming covalent bonds, therefore achieving a non-covalent functionalisation, see P. Bilalis, D. Katsigiannopoulos, A. Avgeropoulos, G. Sakellariou, "Non-covalent functionalization of carbon nanotubes with polymers", RSC ADVANCES, 4, 2911 (2014). Amongst the molecules used, ionic liquids can be cited, as mentioned in Jinyong Wang, Haibin Chu, and Yan Li, "Why Single-Walled Carbon Nanotubes Can Be Dispersed in Imidazolium-Based Ionic Liquids", ACS NANO, 2008, 2(12), 2540-2546.

A typical limit of non-covalent functionalisation is that the interaction between the functional group and the substrate cannot be strong and stable, since it is not based on covalent bonds. Furthermore, the extensive covering of the carbon substrate with the functionalising molecule reduces the possibility of the carbon material transferring its characteristics to whatever surrounds it and this limits the performance and uses thereof. Furthermore, with regard to ionic liquids, limited numbers of these are available and therefore lead to a limited number of possible modifications.

WO 2016/050887 describes an adduct between an sp²-hybridised carbon allotrope and a specific pyrrolic compound, serinol pyrrole. Serinol pyrrole is obtained by means of the Paal Knorr reaction between the serinol and the 2,5-hexandione. Serinol pyrrole is a chemical composition that, in addition to the pyrrole ring, also contains the hydroxyl groups derived from the serinol. Said serinol pyrrole is therefore a so-called Janus molecule, that is a molecule that has two faces and the reactivity of the pyrrole ring and the hydrophilic nature that derives from the serinol. The serinol pyrrole is easily soluble in water. The adduct of the serinol pyrrole with an sp²-hybridised carbon allotrope has increased dispersibility in liquid and polymer matrices that also contain polar groups.

The Doctoral thesis of M. Poikelispää *"Improvements of nanofiller-elastomer systems by filler modification and tailored mixing techniques"* disclosed at the Tampere University disclose nanofillers for elastomes based on surface modified silicates.

WO 2018/087688 disclose adducts formed by primary amines, dicarbonyl derivatives, inorganic oxide hydroxide and sp²-hybridized carbon allotropes.

The preparation of the adducts of serinol pyrrole with an sp²-hybridised carbon allotrope has been described in the following publications: (1) M. Galimberti, V. Barbera, S. Guerra, L. Conzatti, C. Castiglioni, L. Brambilla, A. Serafini, "Biobased Janus molecule for the facile preparation of water solutions of few layer graphene sheets", RSC Adv., 2015, 5, 81142-81152 DOI: 10.1039/C5RA11387C (2) M. Galimberti, V. Barbera, S. Guerra, A. Bernardi, Facile functionalization of sp2 carbon allotropes with a biobased Janus molecule. Accepted for publication on Rubber Chemistry and Technology, 2017, 90(2), 285-307 (3) V. Barbera, A. Bernardi, A. Palazzolo, A. Rosengart, L. Brambilla, M. Galimberti, Pure and Applied Chemistry, 2017 | DOI: https://doi.org/10.1515/pac-2017-0708. From these articles, it seems that the yield of the reaction of functionalising the carbon allotrope with the serinol pyrrole substantially depends on the type of carbon allotrope and can also be very high. Of course, modification with the serinol pyrrole leads to just one type of adduct, which can therefore only have good compatibility with certain types of surrounding agents, mainly of a polar and hydrophilic nature.

In the publication V. Barbera, A. Bernardi, A. Palazzolo, A. Rosengart, L. Brambilla, M. Galimberti, Pure and Applied Chemistry, 2017 | DOI: https://doi.org/10.1515/pac-2017-0708, the adducts having a nanographite with a high surface area are obtained using various pyrrolic compounds. It is shown that, in order to vary the pyrrolic compound, the chemical nature of the adduct varies. The Hansen solubility parameter of an adduct with a pyrrolic compound that yields an alkenyl substituent of the nitrogen atom is calculated. It can be seen that the lipophilic substituent leads to a compatibility with organic solvents, too.

In the industrial sector it is also commonly known to use inorganic oxide hydroxide compositions, for example silica, sheet silicates, mixed oxides consisting of aluminium and magnesium, alumina and silicoaluminates.

Inorganic oxide hydroxides have several industrial applications, for example they are widely used as fillers in the elastomeric material-processing sector.

In this sector, it is known to use precipitated silica as the reinforcing filler as this makes it possible to achieve important advantages, including:
- the reduction in the generation of heat during the use of the tyre, with respect to compounds that contain carbon black as the reinforcing filler, in a temperature range of between 50° and 100 °C, which temperature range is typically reached during the use of the tyres, and
- the improvement of various physical and mechanical properties of the tyres, for example resistance to abrasion and cuts.

These findings are reached despite the chemical and physical nature of the silica that should lead to properties that are quite the opposite. In fact, the presence of oxygenated groups and in particular of hydroxyls leads to important supramolecular interactions, including hydrogen bonds. Taking into consideration the hydrophilic nature thereof and these strong supramolecular interactions, it should be very difficult to distribute and disperse the silica in a lipophilic matrix, such as that of the elastomers used in compounds for tyres. In industrial practice, the silica is chemically modified using a coupling agent, which is typically a silane containing two Si(OR)₃ groups and sulfur atoms The Si(OR)₃ groups bond to the silica while the sulfur atoms encourage bonding of the silica to the elastomer by means of the vulcanisation reaction. It is therefore important to have a filler comprising reactive groups on the surface thereof that enable it to modify chemically in order to encourage the compatibilisation thereof with the polymer matrix.

In this context, the term "adduct" or "super-adduct" denotes a chemical species obtained from two or more chemical species after the establishment of chemical or physical interactions that may be: chemical bonds, such as a covalent bond or an ionic bond, or so-called non-bonded interactions or supramolecular interactions such as Van der Waals forces, ion-dipole interactions and hydrogen bonds.

"Inorganic oxide hydroxide" means an inorganic chemical species comprising an oxide and/or a hydroxide as the functional group.

The term "pyrrolic compound" means a chemical species comprising a pyrrole ring, in which the nitrogen atom preferably bears substituents, these being either lipophilic or hydrophilic substituents. In this context, the term "sp²-hybridised carbon allotrope" or simply "carbon allotrope" denotes a carbon allotrope in which at least some of the carbon atoms that form said carbon allotrope are bonded to one another by means of electronic orbitals that are sp²-hybridised.

First of all, the inventors have proven that sp² carbon allotropes functionalised with pyrrolic compounds, which are not limited to just serinol pyrrole but are obtained, more generally, by reacting a primary amine and a α,δ diketone, not only appear modified in terms of the chemical nature thereof, but demonstrate an increased degree of dispersibility in liquid or polymer matrices that is different for each carbon allotrope, since said matrices are characterised by a different solubility parameter.

The inventors have also observed that the yield of the above reaction for functionalising carbon allotropes with pyrrolic compounds is never quantitative. Therefore, it is possible for the adduct comprising the sp²-hybridised carbon allotrope to lose the pyrrolic compound and to be undesirably dispersed into the reaction/mixing environment. This drawback can result in problems associated with material management, which can prove to be a serious impediment to its application on an industrial scale.

The inventors have also observed that relevant examples of an inorganic oxide hydroxide and of a carbon allotrope, such as silica and carbon black, are used as reinforcing fillers in the same elastomeric composite materials, for example in compounds for tyres. However, the inventors have observed that the two fillers are not bonded to one another by any type of chemical bond.

The inventors have therefore identified that, in order to make the pyrrolic compound easier to manage from an operational point of view, it could be bonded in advance to a chemical species that provides support.

The inventors have therefore found that, by reacting the pyrrolic compounds with an inorganic oxide hydroxide, a stable adduct forms, which can advantageously be used for functionalising an sp²-hybridised carbon allotrope.

The inventors have therefore found that, by reacting this adduct, which is formed of a pyrrolic compound and an inorganic oxide hydroxide, with an sp²-hybridised carbon allotrope, a super-adduct having a stable interaction is formed.

Therefore, in a first aspect thereof, the present invention relates to an adduct according to claim 1.

The adduct obtained in this way has a stable interaction between the inorganic oxide hydroxide and the pyrrolic compound and, on account of this characteristic, the adduct substantially does not release the pyrrolic compound, namely losses of the pyrrolic compound from said adduct are substantially avoided. This advantageously allows the pyrrolic compound that forms the adduct to also be used in a simple and reliable manner in any subsequent industrial applications.

In addition, the structure of the inorganic oxide hydroxide confers excellent mechanical strength properties to the adduct.

In order to investigate the nature of the stable interaction that bonds the inorganic oxide hydroxide and the pyrrolic compound more effectively, the inventors have therefore subjected an adduct comprising serinol pyrrole (as the pyrrolic compound) and silica (as the inorganic oxide hydroxide) to a series of analyses.

In particular, analysis of the adduct by means of infrared spectroscopy by attenuated total reflectance (ATR) has shown bands characteristic of the pyrrole ring. It is therefore possible to hypothesize that the pyrrole ring of the serinol pyrrole does not lose its aromatic nature, but it can be found in the adduct with the silica, even after having been subjected to difficult reaction conditions, such as a reaction at 150 °C in the absence of solvents.

It is also possible to observe the onset of an asymmetry of the signals of the pyrrole ring in the infrared spectrum of the adduct between the silica and the serinol pyrrole. This makes it possible to hypothesize that there exists an interaction between the pyrrole ring and the silica that could lead to the modification of the signals in the infrared spectrum. This interaction could also be a chemical bond.

Furthermore, the adduct improves the dispersibility of the inorganic oxide hydroxide inside matrices, for example liquid (having a low or high viscosity) or polymer matrices, on account of the suitable selection of the substituent of the nitrogen atom of the pyrrole ring.

Furthermore, the inventors have found that the adduct obtained in this way is able to functionalise another chemical species, substantially acting as an intermediate stage of the functionalisation process.

In particular, by reacting the adduct with a carbon allotrope, a super-adduct is obtained that displays a stable interaction between the sp²-hybridised carbon allotrope and the silica, from which adduct the release of the pyrrolic compound is not substantially noticeable. This super-adduct considerably improves the dispersibility characteristics of the carbon allotrope in a liquid or a polymer blend as a result of the appropriate selection of the pyrrolic compound and the quantity of inorganic oxide hydroxide. This liquid and this polymer blend can have a different solubility parameter.

In a second aspect thereof, the present invention relates to a first method for preparing an adduct according to the preceding aspect in accordance with claim 7.

In a third aspect thereof, the present invention relates to a second method for preparing an adduct according to the first aspect in accordance with claim 8.

This process makes it possible to obtain the adduct directly from the reagents of the pyrrolic compound, without having to handle or manage the pyrrolic compound which in fact bonds to the inorganic oxide hydroxide as soon as it has formed.

In this way, any potential problems associated with handling the pyrrolic compound are avoided.

In a fourth aspect thereof, the present invention relates to a super-adduct according to claim 13.

In this way, the carbon allotrope is functionalised, which can increase the dispersibility thereof in liquid or polymer matrices that have a different solubility parameter. In fact, the presence of the inorganic oxide hydroxide in the super-adduct makes it possible to increase the polarity of the sp²-hybridised carbon allotrope. The amount of inorganic oxide hydroxide can make it possible to modulate the polarity of the super-adduct and therefore the dispersibility in matrices having different polarities. Furthermore, the use of different pyrrolic compounds makes it possible to modulate the solubility parameter of the sp²-hybridised carbon allotrope and therefore the dispersibility in matrices that have a different solubility parameter.

Furthermore, the formation of the super-adduct does not result in any loss of the pyrrolic compound, thus leading to speculation that the carbon allotrope is functionalised as a result of a stable interaction between the allotrope and the pyrrolic compound, for example.

In particular, it has been proven that this super-adduct does not release substances used for the functionalisation process either following treatment with solvents or following thermomechanical treatment, at least up to a temperature of approximately 100 °C, preferably up to a temperature of approximately 150 °C.

In a fifth aspect thereof, the present invention relates to a method for preparing a super-adduct according to the fourth aspect, in accordance with claim 14.

The inventors have verified that this method for producing the super-adduct makes it possible to functionalise carbon allotropes without having to provide dedicated equipment and/or plants, but by using technology that already exists, substantially technology used for mixing polymer materials.

Also, it has been verified that this method for producing the super-adduct can be defined as a versatile method, specifically that makes it possible to easily introduce different types of functional groups on the carbon allotrope.

In a sixth aspect, the present invention relates to an elastomeric composition according to claim 15.

The inventors have in fact verified that these compositions have an optimum combination of dynamic mechanical properties, tensile properties and a reduction in energy dispersion, which render said compositions particularly suitable for their use in producing compounds for tyres.

In at least one of the above-mentioned aspects, the present invention can have at least one of the preferred characteristics indicated below.

Said ratio between the weight of said inorganic oxide hydroxide and said pyrrolic compound is preferably greater than or equal to 50, more preferably greater than or equal to 100, and even more preferably greater than or equal to 500.

In a preferred embodiment, Y and Z are independently selected from said second group; W is selected from said first group; R₂₀, R₂₁, R₂₅, R₂₆, R₂₈, R₂₉, R₃₁ and R₃₂ are independently selected from the group consisting of: hydrogen, C₁-C₁₈ alkyl and linear or branched C₂-C₁₈ alkenyl or alkynl; and R₂₂, R₂₃, R₂₄, R₂₇, R₃₀, R₃₃, R₃₄, R₃₅, R₃₆ are independently selected from the group consisting of: hydrogen, C₁-C₁₈ alkyl, linear or branched C₂-C₁₈ alkenyl or alkynl, aryl, linear or branched C₁-C₂₂ alkyl-aryl, linear or branched C₂-C₂₂ alkenyl-aryl, linear or branched C₂-C₂₂ alkynl-aryl, heteroaryl and carboxyl.

In another preferred embodiment, Y is selected from said second group; W and Z are independently selected from said first group; R₂₀, R₂₁, R₂₅, R₂₆, R₂₈, R₂₉, R₃₁, R₃₂ are independently selected from the group consisting of: hydrogen, C₁-C₁₈ alkyl and linear or branched C₂-C₁₈ alkenyl or alkynl; and R₂₂, R₂₃, R₂₄, R₂₇, R₃₀, R₃₃, R₃₄, R₃₅, R₃₆ are independently selected from the group consisting of: hydrogen, C₁-C₁₈ alkyl, linear or branched C₂-C₁₈ alkenyl or alkynl, aryl, linear or branched C₁-C₂₂ alkyl-aryl, linear or branched C₂-C₂₂ alkenyl-aryl, linear or branched C₂-C₂₂ alkynl-aryl, heteroaryl and carboxyl.

The N-R group in the compound of formula I preferably derives from the following primary amine: methylamine, 1-hexylamine, 1-dodecylamine, 1-octadecylamine, 2-amino-1,3-propanediol (serinole), 2-aminoethanol, 2-aminoacetic acid, 3-triethoxysilyl-propan-1-amine, 3-(diethoxyl(methoxy)silyl)propan-1-amine, *N',N'*-dimethylpropan-1,3-diamine, 4,4'-methylene dicyclohexanamine, 3-(aminomethyl)-3,5,5-trimethylcyclohexanamine, *O,O'* - *b*is(2-aminpropyl)propylene.

According to a preferred embodiment, said pyrrolic compound is selected from the group formed of 2,5-dimethyl-*N*-hexylpyrrole (hexylpyrrole, EP), 2,5-dimethyl-*N*-dodecyl pyrrole (DDcP), 1-(3-(triethoxysilyl)propyl)-2,5-dimethyl-1*H*-pyrrole, 2-(2,5-dimethyl-1*H*-pyrrol-1-yl)acetic acid, 2-pyrrol-1-yl-1,3-propandiol, 1,2,5-trimethylpyrrole and serinol pyrrole (SP).

According to the most preferred embodiment, said pyrrolic compound is serinol pyrrole.

On account of this technical solution, the pyrrolic compound is optimally bonded to the inorganic oxide hydroxide, thus demonstrating high and ideal chemical stability.

The applicant has in fact proven that the serinol pyrrole forms a very stable adduct together with the inorganic oxide hydroxide as a result of a stable interaction, which could be a chemical bond, between the serinol pyrrole and the inorganic oxide hydroxide.

In a preferred version, said inorganic oxide hydroxide is amorphous silica. The amorphous silica can be a naturally occurring rubber, such as diatomite, or synthetic.

Precipitated silica, silica gel, fumed silica or silica subjected to superficial treatments may be used as synthetic silicas.

The silica can be fumed or, preferably, precipitated, having a BET surface area of between 50 m²/g and 500 m²/g, preferably between 70 m²/g and 200 m²/g.

According to one embodiment, said inorganic oxide hydroxide is a sheet silicate selected from the group consisting of serpentine-kaolin, talc-pyrophyllite, smectite, vermiculite, mica, chlorites, palygorskite and sepiolite, allophane and imogolite.

Said inorganic oxide hydroxide is preferably a sheet silicate in which each individual sheet has a thickness of between 0.1 and 30 nm, preferably between 0.5 and 15 nm, and even more preferably between 0.8 and 2 nm.

Said inorganic oxide hydroxide is most preferably a sheet silicate selected from the group consisting of montmorillonite, bentonite, beidellite, nontronite, volkonskoite, hectorite, fluoroectorite, laponite, saponite, stevensite, sauconite, vermiculite, mica, celadonite, lepidolite, muscovite, phlogopite, chlorite, palygorskite, sepiolite, allophane, imogolite and hydrotalcite.

Due to the above-mentioned features, it is possible to reach an ideal technical solution that makes it possible to achieve a high degree of dispersibility of the adduct in the liquid or polymer matrix together with a significant degree of chemical and thermal stability.

The sheet inorganic oxide hydroxides can be anionic: only hydrotalcites are naturally occurring.

The sheet inorganic oxide hydroxides can be naturally occurring or synthetic.

These sheet materials (known in international scientific literature as clay) are a subset of the family of sheet oxides and are usually classified into three different categories depending on the electric charge of the layer: (i) neutral layer, (ii) negatively charged layer and (iii) positively charged layer.

The sheet materials having a neutral layer or a negatively charged layer can belong to the group of serpentine and kaolin, to the group of talc and pyrophyllite, to the group of smectites, such as montmorillonite, bentonite, beidellite, nontronite, volkonskoite, hectorite, fluorohectorite, laponite, saponite, stevensite, sauconite to the group of vermiculite, to the group of mica, such as celadonite, lepidolite, muscovite, phlogopite, to the group of chlorites, to the group of palygorskite e sepiolite, to the group of allophane and of imogolite. The sheet materials having a positively charged layer can in particular be those of the group of hydrotalcite.

According to a preferred embodiment, the method for preparing said adduct provides that said energy supplied to the reaction mixture is thermal energy and/or mechanical energy and/or energy for irradiation with photons.

According to a preferred embodiment, the method for preparing said adduct provides that said pyrrolic compound is oxidised before being mixed with said inorganic oxide hydroxide.

Due to this technical solution, it is possible to further increase the efficiency and yield of the above-mentioned method.

Said pyrrolic compound is preferably oxidised by an oxygen-donor composition selected from the group consisting of: H₂O₂, MnO₂, KMnO₄, HNO₃, NaClO (sodium hypochlorite), NaClO₂, H₂SO₄/HNO₃, NaIO₄, HIO₄, OsO₄, Pb(C₂H₃O₂)₄, HNO₃/HNO₂, CO₂, CO, dicumyl peroxide, RuO₂, RuO₄, SeO₂, O₃, periodinane, peracetic acid, iodobenzoic acid, nicotinamide adenine dinucleotide (NAD+) and laccase or oxidase enzymes.

Alternatively, said pyrrolic compound is oxidised by the oxygen present in the reaction environment.

The pyrrolic compound can preferably be oxidised in the following ways:
(i) said pyrrolic compound is placed in a solvent, preferably selected from alcohols, chetones, ethers, esters, and more preferably being water,
(ii) oxygen or one of said oxygen-donor compositions is added,
(iii) the mixture thus formed is stirred at a predetermined temperature, preferably at a temperature of between 20 °C and the reflux temperature of the solvent, and for a preset time, preferably of between 1.5 and 3 hours.

In a first embodiment, the amounts by weight are mixed and energy is supplied in the presence of at least one solvent. This technical solution makes it possible to use the solvent as a thermal flywheel, thereby optimising the reaction and ensuring uniform reactivity between the reagent species.

In this case, the method may provide:
(i) adding the pyrrolic compound to said solvent,
(ii) adding said inorganic oxide hydroxide to the solution/dispersion prepared in (i),
(iii) mixing, using a magnetic or mechanical stirrer,
(iv) stirring using a magnetic or mechanical stirrer in order to form said adduct, and
(v) isolating said adduct by eliminating the solvent by means of evaporation or filtration.

The mixture is preferably stirred in a temperature range of between 0 °C and the reflux temperature of the solvent, more preferably between 15 °C and 50 °C.

The mixture is preferably stirred for between 5 minutes and 6 hours, more preferably between 10 minutes and 2 hours, and even more preferably between 30 minutes and 1 hour.

The at least one solvent is preferably selected from eco-compatible solvents.

The at least one solvent is more preferably selected from the group consisting of alcohols, aldehydes, chetones, esters, ethers and water.

According to the most preferred embodiment, the at least one solvent is water-based.

According to an alternative embodiment, energy is supplied in the absence of solvents.

This technical solution preferably results in a viable chemical standpoint, thus also facilitating and simplifying the storage conditions of the reagents.

In this case, the method may provide:
(i) adding said pyrrolic compound to said solvent,
(ii) adding said inorganic oxide hydroxide to the solution/dispersion prepared in (i),
(iii) mixing using a magnetic or mechanical stirrer,
(iv) stirring using a magnetic or mechanical stirrer in a temperature range of from 15 °C to 30 °C for between 1 and 10 minutes,
(v) removing the solvent by means of evaporation or filtration,
(vi) heat treating at a temperature of between 0 °C and 200 °C, preferably between 10 °C and 150 °C, more preferably between 20 °C and 100 °C, or thermomechanical treatment carried out using instruments such as mortars, ball mills or rod mills, in a temperature range of from 10 °C to 50 °C.

According to another alternative embodiment, the amounts by weight are mixed and energy is supplied in the absence of solvents.

According to one embodiment, said carbon allotrope is nano-carbon or nano-structured.

In this way, it is possible to noticeably increase the electrical and thermal conductivity of the matrix in which the super-adduct is dispersed, for example.

Said carbon allotrope is preferably selected from a group consisting of graphite, nanographites consisting of a few graphene layers (from a few units to several tens of units), graphene, carbon black (lampblack), fullerene, carbon nanotubes having one or more walls, nanohorns, nanographites, nanotoroids, nanobelts, and the like.

Even more preferably, the carbon allotrope is selected from the group consisting of carbon black, fullerene, carbon nanotubes having one or more walls, graphene, graphite having between 2 and 10000 graphene layers.

The carbon allotrope may contain functional groups selected from the group consisting of:
- oxygenated functional groups, preferably hydroxyls, epoxides;
- functional groups containing carbonyls, preferably aldehydes, chetones, carboxyl acids;
- functional groups containing nitrogen atoms, preferably amines, amides, nitriles, diazonium salts, imines; and
- functional groups containing sulfur atoms, preferably sulfurs, dissulfurs, mercaptanes, sulfones, sulfinic or sulfonic groups.

In this way, a wide range of carbon allotropes is provided.

The carbon allotrope derivative is preferably graphite oxide or graphene oxide. Sp²-hybridised carbon allotropes are found to be advantageous for various desired characteristics and applications. The electric and thermal conductivity, for example of composite materials that would not require the presence of fillers or which require the presence of non-conductive fillers (for example silica), are obtained by means of "nano" allotropes, such as nanotubes, nanographites and graphenes, which are able to create lattices of continuous filler, thus offering electric and thermal conductivity while keeping the amount of allotrope to a minimum. Various types of allotropes are used to support the mechanical reinforcement of composite materials. Attempts have been made to create an optimum interaction between the allotropes and the matrix, an interaction that has to be stable under the use conditions of the material, thereby minimising and, if possible, preventing interaction between allotrope particles and aggregates. In this way, the properties of the allotrope are transferred to the matrix, preventing the *"filler networking"* phenomenon.

According to a preferred embodiment, the ratio between said fourth amount by weight and said third amount by weight is between 1 and 1000, more preferably between 2 and 200, and even more preferably between 10 and 100.

On account of this technical solution, the super-adduct comprising the carbon allotrope and the polymer or liquid matrix have solubility parameters that are as similar as possible so as to obtain effective and stable dispersions.

According to a preferred embodiment, the method for preparing said super-adduct provides that said energy supplied to the reaction mixture is thermal and/or mechanical energy and/or energy for irradiation with photons.

In a preferred version, the method for preparing said super-adduct provides:
(i) mixing said adduct with said carbon allotrope, and
(ii) obtaining said super-adduct by heat-treating or thermomechanically treating the mixture obtained in this way.

Said heat treatment is preferably carried out within a temperature range of between 50 °C and 200 °C, preferably between 75 °C and 180 °C, even more preferably between 100 °C and 150 °C, for between 5 minutes and 6 hours, preferably between 10 minutes and 3 hours, and more preferably between 30 minutes and 2 hours.

Said thermomechanical treatment is preferably carried out using instruments such as mortars, ball mills or rod mills, in a temperature range of from 10 °C to 50 °C.

In a preferred embodiment, the elastomeric composition according to one aspect of the present invention comprises at least one elastomeric diene polymer. The elastomeric diene polymer can be selected from those that are normally used in elastomeric sulfur-crosslinked compositions. This elastomeric diene polymer contains unsaturations in the polymer chain and has a glass transition temperature (Tg) that is generally lower than 20 °C, preferably between 0 °C and -110 °C. Said diene elastomeric polymer can be naturally occurring or produced in a reactor for synthesising polymers, using a technology that operates in a solution or in an emulsion or in a gas phase. The monomers that are polymerised are one or more conjugated diolefins, optionally mixed with at least one comonomer selected from monovinylarenes and/or polar comonomers having from 8 to 20 carbon atoms. The amount of comonomer selected from monovinylarenes and/or polar comonomers that have from 8 to 20 carbon atoms is preferably no more than 60 wt.%.

The conjugated diolefin has between 4 and 12 carbon atoms, preferably between 4 and 8 carbon atoms, and may be selected from a group consisting of: 1,3-butadiene, isoprene, 2,3-dimethyl-1,3-butadiene, 1,3-pentadiene, 1,3-hexadiene, 3-butyl-1,3-octadiene, 2-phenyl-1,3-butadiene, or mixtures thereof, for example. 1,3-butadiene and isoprene are particularly preferred.

The monovinylarenes, which can optionally be used as comonomers, generally contain from 8 to 20 carbon atoms, preferably from 8 to 12 carbon atoms, and can be selected from: styrene, 1-vinylnaphthalene; 2- vinylnaphthalene; various alkyls, cycloalkyls, aryls, alkylaryls or aylalkyl derivatives of styrene, such as 1-methylstyrene, 3-methylstyrene, 4-propylstyrene, 4-cyclohexylstyrene, 4-dodecylstyrene, 2-ethyl-4-benzylstyrene, 4-p-tolylstyrene, 4-(4-phenylbutyl)styrene, or mixtures thereof. Styrene is preferred.

The polar comonomers that can optionally be used are selected from: vinylpyridine, vinylquinoline, acrylic acid and esters of acrylic acid, nitriles or mixtures of these comonomers, for example. Examples of acrylic esters are: methyl acrylate, ethyl acrylate, methyl methacrylate, ethyl methacrylate and acrylonitrile.

The elastomeric diene polymer is preferably selected from the group consisting of: poly(1,4-cis-isoprene), both natural rubber and synthetic polymer, poly(3,4-isoprene), poly(butadiene) (in particular poly(butadiene) having a high content of 1,4-cis units), optionally halogenated isoprene/isobutene copolymers such as halogenated butyl rubber, in particular chlorobutyl rubber and bromobutyl rubber, copolymers 1,3-butadiene/acrylonitrile, copolymers styrene/1,3-butadiene, copolymers styrene/isoprene/1,3-butadiene, copolymers styrene/1,3-butadiene/acrylonitrile, or mixtures of these polymers. The elastomeric composition according to one aspect of the present invention can also contain at least one elastomer of one or more mono-olefins. The mono-olefins can be selected from: ethylene and 1-olefin that contain from 3 to 12 carbon atoms, such as propylene, 1-butene, 1-pentene, 1-hexene, 1-octene, or mixtures of these mono-olefins.

The elastomer of one or more mono-olefins can contain a diene, which generally contains from 4 to 20 carbon atoms and is preferably selected from: 1,3-butadiene, isoprene, 1,4-hexadiene, 1,4-cyclohexadiene, 5-ethylidene-2-norbornene, 5-methylene-2-norbornene, vinyl norbornene or mixtures of these dienes. The diene can optionally be halogenated.

Of these elastomers of one or more mono-olefins, the following are preferred: ethylene/propylene (EPR) copolymers or ethylene/propylene/diene (EPDM) copolymers and poyl(isobutene).

The elastomeric composition according to one aspect of the present invention can also contain a diene elastomer or an elastomer based on non-diene monomers, which is functionalised by reaction with a suitable termination agent or coupling agents. In particular, the elastomeric diene polymer can be obtained by means of anionic polymerisation that is promoted by an organometallic initiator (in particular an alkyllithium initiator), and terminated by reaction with suitable termination agents or coupling agents, such as epoxides, carbonyl compounds such as substituted or unsubstituted cyclohexanone and benzophenone, immines, carbodiimides, halides of alkyltins, alkoxylsilanes and aryloxysilanes.

A few detailed examples of the preparation of adducts and super-adducts in accordance with the present invention will be discussed in detail in the following.

### EXAMPLES

### Materials

The graphite used is Synthetic Graphite 8427, purchased from Asbury Graphite Mills Inc., which has a minimum carbon content equal to 99.8 wt.% and a surface area equal to 330 m²/g. The multi-walled carbon nanotubes (MWCNT) used are the NC7000 series by NANOCYL Inc. The carbon black used is the Carbon Black N326 (Cabot), which has the following features: 30 nm average diameter of the spherical particles, a surface area equal to 77 m²/g (determined by means of nitrogen absorption), DBP absorption equal to 85 ml/100 g.

Another carbon black used is Carbon Black N234 (Cabot), which has the following features: 152 nm average diameter of the spherical particles, surface area equal to 1182 m²/g (determined by means of nitrogen absorption).

The serinol pyrrole is synthesised in the manner described in M. Galimberti, V. Barbera, S. Guerra, L. Conzatti, C. Castiglioni, L. Brambilla, A. Serafini, "Biobased Janus molecule for the facile preparation of water solutions of few layer graphene sheets", RSC ADV., 2015, 5, 81142-81152. The 1,2,5-trimethyl-pyrrole used in Example 13 was purchased from Aldrich.

### EXAMPLES 1 - 14 and 1bis-4bis

### Preparing adducts between the inorganic oxide hydroxide and the non-oxidised pyrrolic compound

### EXAMPLES 1-5

### Synthesis of pyrrolic compounds

### Example 1

### Synthesis of 2,5-dimethyl-N-hexylpyrrole (hexylpyrrole, EP)

2,5-hexanedione (1.69 g, 14.8 mmol) and 1-hexaneamine (1.5 g, 14.8 mmol) are sequentially poured into a 100-ml flask equipped with a magnetic stirrer. The mixture obtained in this way is stirred for 3 hours at 130 °C. At this point, the reaction mixture is left to cool down to room temperature and the reaction water is then removed under reduced pressure by means of a rotary evaporator. The product 2,5-dimethyl-*N*-hexylpyrrole (HP) is isolated and analysed by means of NMR spectroscopy. The product obtained is in the form of an amber oil. After weighing, taking into account the purity of the composition detected by the NMR, a yield equal to 73 % is estimated.

### Example 2

### Synthesis of 2,5-dimethyl-N-dodecyl pyrrole (DDcP)

2,5-hexanedione (1.69g, 14.8 mmol) and 1-dodecaneamine (3.9 g, 14.8 mmol) are sequentially poured into a 100-ml flask equipped with a magnetic stirrer. The mixture obtained in this way is stirred for 3 hours at 130 °C. At this point, the mixture is left to cool down to room temperature and the water produced by the reaction is then removed under reduced pressure by means of a rotary evaporator. The product 2,5-dimethyl-*N*-dodecyl pyrrole (DDcP) is in the form of an amber oil. It is analysed by means of NMR spectroscopy: the only composition detected is 2,5-dimethyl-N-dodecyl pyrrole. After weighing, taking into account the purity of the composition detected by the NMR, a yield equal to 62 % is estimated.

### Example 3

### Synthesis of 1-(3 -(triethoxysilyl)propyl)-2,5-dimethyl-1H-pyrrole

2,5-hexanedione (0.55 g, 4.82 mmol) and 3-(triexthoxylsilyl)propane-1-amine (1 g, 4.82 mmol) are sequentially poured into a 100-ml flask equipped with a magnetic stirrer. The mixture obtained in this way is stirred for 3 hours at 130 °C. At this point, the mixture is left to cool down to room temperature and the reaction water is then removed under reduced pressure by means of a rotary evaporator. The product 1-(3-(triethoxysilyl)propyl)-2,5-dimethyl-1H- pyrrole (APTESP) is isolated and analysed by means of NMR spectroscopy. The product obtained is in the form of an amber oil. After weighing, taking into account the purity of the composition detected by the NMR, a yield equal to 80 % is estimated.

### Example 4

### Synthesis of 2-(2,5-dimethyl-1H-pyrrol-1-yl)acetic acid

2,5-hexanedione (1.52 g, 1.85 mmol) and 2-aminoacetic acid (1 g, 1.85 mmol) are sequentially poured into a 100-ml flask equipped with a magnetic stirrer. The mixture obtained in this way is stirred for 3 hours at 130 °C. At this point, the mixture is left to cool down to room temperature and the reaction water is then removed under reduced pressure by means of a rotary evaporator. The product 2-(2,5-dimethyl-1H-pyrrol-1-yl)acetic acid (GliP) is isolated and analysed by means of NMR spectroscopy. The product obtained is in the form of an amber oil. After weighing, taking into account the purity of the composition detected by the NMR, a yield equal to 80 % is estimated.

### Example 5

### Synthesis of 2-pyrrol-1-yl-1,3-propandiol

1.32 g of 2,5-dimethoxyltetrahedrofuran (10 mmol), 1 g of serinol (11 mmol) and 50 ml of HCl 0.1 N (5 mmol) are measured at reflux for one night in a 100-ml flask having a neck equipped with a magnetic reflux stirrer. At the end of the reaction, the pH of the solution is neutralised by adding NaHCO₃ and the solvent is moved from the reaction mixture to the rotary evaporator. The residue is therefore dissolved in ethyl acetate several times in order to extract the reaction product. After weighing, taking into account the purity of the composition detected by the NMR, a yield equal to 92 % is estimated.

### EXAMPLES 6-14

### Preparation of adducts between the inorganic oxide hydroxide and the pyrrolic compound from the pyrrolic compounds in Examples 1-5 and from trimethylpyrrole

### Example 6

Preparation of silica adduct with serinol pyrrole 10 g of Zeosil 1165 silica are poured into a 250 ml-flask equipped with a magnetic stirrer, and 15 ml of acetone are added. The mixture is sonicated for 10', then 1 g of serinol pyrrole is added as well as another 10 ml of acetone. The mixture is sonicated for another 15 minutes. At this point, the solvent is removed under reduced pressure and the mixture is stirred by means of the magnetic stirrer for 2 hours at a temperature of 150 °C. At the end of the reaction, the mixture is left to cool down to room temperature. The product is in the form of an ochre-coloured powder.

### Example 7

Preparation of silica adduct with hexylpyrrole 10 g of silica are poured into a 250 ml-flask containing 15 ml of acetone. The mixture is sonicated for 10', then 1 g of hexylpyrrole, as prepared in Example 1, is added as well as another 10 ml of acetone, and the mixture is sonicated for another 15'. At this point, the solvent is removed under reduced pressure and the flask is magnetically stirred at a temperature of 150 °C for 2 hours. At the end of the reaction, the mixture is left to cool down to room temperature and the product that is in the form of a brick-coloured powder is retrieved.

### Example 8

Preparation of silica adduct with 2,5-dimethyl-N-dodecylpyrrole 10 g of silica are poured into a 250 ml-flask containing 15 ml of acetone. The mixture is sonicated for 10', then 1 g of 2,5-dimethyl-N-dodecylpyrrol, as prepared in Example 2, is added as well as another 10 ml of acetone, and the mixture is sonicated for another 15'. At this point, the solvent is removed under reduced pressure and the flask is magnetically stirred at a temperature of 150 °C for 2 hours. At the end of the reaction, the mixture is left to cool down to room temperature and the product that is in the form of a dark-orange powder is retrieved.

### Example 9

Preparation of silica adduct with 1-(3-(triethoxysilyl)propyl)-2,5-dimethyl-1H-pyrrole 10 g of silica are poured into a 250 ml-flask containing 15 ml of acetone. The mixture is sonicated for 10', then 1 g of 1-(3-(triethoxysilyl)propyl)-2,5-dimethyl-1H-pyrrole, as prepared in Example 3, is added as well as another 10 ml of acetone, and the mixture is sonicated for another 15'. At this point, the solvent is removed under reduced pressure and the flask is magnetically stirred at a temperature of 150 °C for 2 hours. At the end of the reaction, the mixture is left to cool down to room temperature and the product that is in the form of a dark-orange powder is retrieved.

### Example 10

Preparation of silica adduct with 2-(2,5-dimethyl-1H-pyrrol-1-yl)acetic acid 10 g of silica are poured into a 250 ml-flask containing 15 ml of acetone. The mixture is sonicated for 10', then 1g of 2-(2,5-dimethyl-1H-pyrrol-1-yl)acetic acid, as prepared in Example 4, is added as well as another 10 ml of acetone, and the mixture is sonicated for another 15'. At this point, the solvent is removed under reduced pressure and the flask is magnetically stirred at a temperature of 150 °C for 2 hours. At the end of the reaction, said mixture is left to cool down to room temperature and the product that is in the form of a dark-orange powder is retrieved.

### Example 11

Preparation of sepiolite adduct with serinol pyrrole 5 g of sepiolite are placed in a 100-ml flask equipped with a magnetic stirrer, and 15 ml of acetone are added. The mixture is sonicated for 10', then 0.5 g of serinol pyrrole are added as well as another 10 ml of acetone. The mixture is sonicated for another 30 minutes. At this point, the solvent is removed under reduced pressure and the mixture is stirred, using the magnetic stirrer, for 2 hours at a temperature of 150 °C. At the end of the reaction, said mixture is left to cool down to room temperature. The product is in the form of an ochre-coloured powder.

### Example 12

Preparation of montmorillonite adduct with serinol pyrrole 5 g of montmorillonite are placed in a 100-ml flask equipped with a magnetic stirrer, and 15 ml of acetone are added. The mixture is sonicated for 10', then 0.5 g of serinol pyrrole is added as well as another 10 ml of acetone. The mixture is sonicated for another 30 minutes. At this point, the solvent is removed under reduced pressure and the mixture is stirred, using the magnetic stirrer, for 2 hours at a temperature of 150 °C. At the end of the reaction, said mixture is left to cool down to room temperature. The product is in the form of a brick-red powder.

### Example 13

Preparation of silica adduct with 1,2,5-trimethylpyrrole 10 g of silica are poured into a 250 ml-flask containing 15 ml of acetone. The mixture is sonicated for 10 minutes, then 0.6 g of 1,2,5-trimethylpyrrole is added as well as another 10 ml of acetone, and the mixture is sonicated for another 15 minutes. At this point, the solvent is removed under reduced pressure and the flask is magnetically stirred at a temperature of 150 °C for 2 hours. At the end of the reaction, said mixture is left to cool down to room temperature and the product that is in the form of a pale-amber powder is retrieved.

### Example 14

Preparation of silica adduct with 2-pyrrol-1-yl-1,3-propanediol 10 g of silica are poured into a 250 ml-flask containing 15 ml of acetone. The mixture is sonicated for 10', then 1 g of 2-pyrrol-1-yl-1,3-propanediol, as prepared in Example 5, is added as well as another 10 ml of acetone, and the mixture is sonicated for another 15'. At this point, the solvent is removed under reduced pressure and the flask is magnetically stirred at a temperature of 150 °C for 2 hours. At the end of the reaction, said mixture is left to cool down to room temperature and the product that is in the form of a pale-amber powder is retrieved.

### EXAMPLES 1bis - 4bis

### Preparation of silica adduct with serinol pyrrole (SP) from the reagents of the SP

### Example 1bis

2,000 g of Zeosil 1165 silica, 0.303 g of serinol and 0.380 g of 2,5-hexanedione are sequentially placed in a 250-ml flask equipped with a magnetic stirrer. The mixture is stirred (400 rpm) for 7 days at room temperature. The product is in the form of an ochre-coloured powder.

### Example 2bis

2,000 g of Zeosil 1165 silica, 0.303 g of serinol and 0.380 g of 2,5-hexanedione are sequentially placed in a 250-ml flask equipped with a magnetic stirrer. The mixture is left for 2 hours at a temperature of 150 °C. At the end of the reaction, the system is left to cool down to room temperature. The product is in the form of an ochre-coloured powder.

### Example 3bis

2000 g of silica and 0.303 g of serinol are placed in 25 ml of ethyl acetate in a 100-ml flask.

In another 100-ml flask, 2 g of silica and 0.380 g of 2,5-hexanedione are placed in 25 ml ethyl acetate. Each flask is sonicated for 30 minutes and the solvents are successively evaporated by reducing the pressure.

The two mixtures are then combined in a single 250-ml flask equipped with a magnetic stirrer. The system is stirred (200 rpm) for 7 days at room temperature.

### Example 4bis

2000 g of silica and 0.303 g of serinol are placed in 25 ml of ethyl acetate in a 100-ml flask.

In another 100-ml flask, 2 g of silica and 0.380 g of 2,5-hexanedione are placed in 25 ml ethyl acetate. Each flask is sonicated for 30 minutes and the solvents are successively evaporated by reducing the pressure.

The two mixtures are then combined in a single 250-ml flask equipped with a magnetic stirrer. The system is stirred (200 rpm) for 2 hours at a temperature of 150 °C.

### EXAMPLES 15-19

### Preparation of adducts between the inorganic oxide hydroxide and the pyrrolic compound from the oxidised pyrrolic compound

### EXAMPLES 15-16

### Preparation of oxidised pyrrolic compounds

### Example 15

### Synthesis of oxidised serinol pyrrole

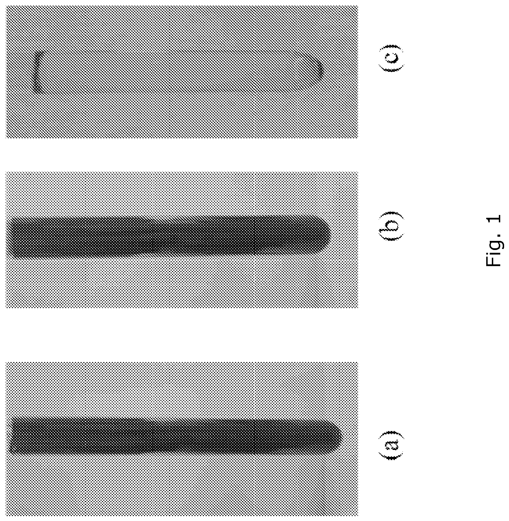

The following are sequentially poured into a 50-ml flask equipped with a magnetic stirrer and a refrigerant: serinol pyrrole (1 g, 0.0059 mol), H₂O (10 ml) and H₂O₂ (30 % v/v, 0.18 ml, 0.0059 mol). The mixture is left to react at 70 °C for 12 hours while being magnetically stirred. At the end of the reaction, the solvent is removed under reduced pressure by means of a rotary evaporator. The oxidised product of the 2-(2,5-dymethyl-1H-pyrrol-1-yl)1,3-propanediol (SP) obtained in this way is a red oil and is analysed by means of NMR spectroscopy. The NMR spectrum is shown in Fig. 1. In the zone between approximately 8 and 9 ppm of the NMR spectrum, peaks can be seen that are compatible with the presence of oxidised species.

### Example 16

### Synthesis of oxidised 1,2,5-trimethylpyrrole

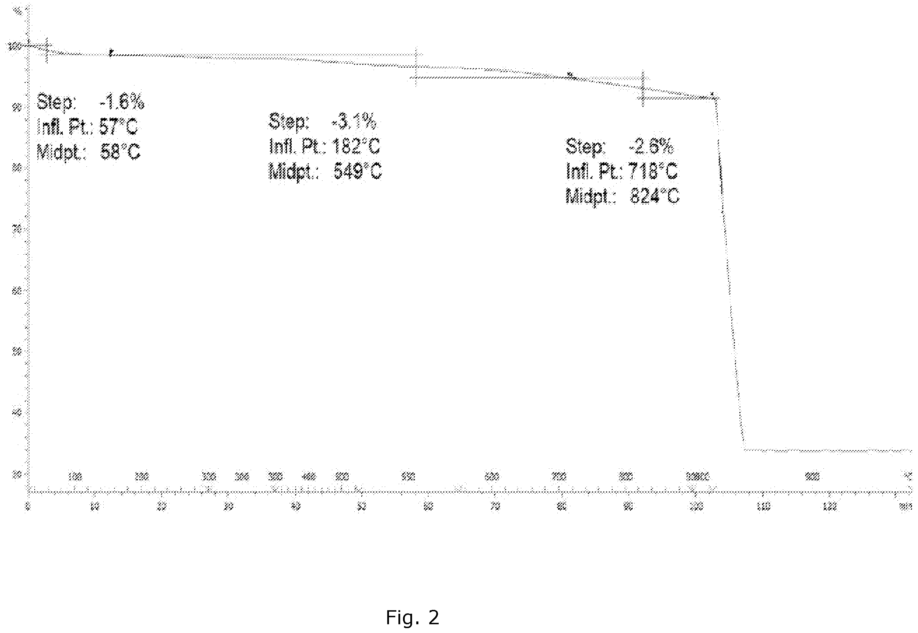

The following are sequentially poured into a 50-ml flask equipped with a magnetic stirrer and a refrigerant: 1,2,5-trimethylpyrrole (0.93 g, 8.60 mmol), H₂O (10 ml) and H₂O₂ (30 % v/v, 0.78 ml, 8.60 mmol). The mixture is left to react at room temperature for 24 hours while being magnetically stirred. At the end of the reaction, the solvent is removed under reduced pressure by means of a rotary evaporator. The oxidised product of the 1,2,5-trimethylpyrrole (TMPO) obtained in this way is a dark-red oil and is analysed by means of NMR spectroscopy. The NMR spectrum is shown in Fig. 2. In the zone between approximately 8 and 9 ppm of the NMR spectrum, peaks can be seen that are compatible with the presence of oxidised species.

### EXAMPLES 17-19

### Preparation of adducts between the inorganic oxide hydroxide and oxidised pyrrolic compounds

### Example 17

Preparation of silica adduct with oxidised serinol pyrrole 10 g of Zeosil 1165 silica are placed in a 250-ml flask equipped with a magnetic stirrer, and 15 ml of acetone are added. The mixture is sonicated for 10', then 1 g of oxidised serinol pyrrole, as in Example 15, is added as well as another 10 ml of acetone. The mixture is sonicated for another 30 minutes. At this point, the solvent is removed under reduced pressure and the mixture is stirred, using the magnetic stirrer, for 2 hours at a temperature of 150 °C. At the end of the reaction, said mixture is left to cool down to room temperature. The product is in the form of a pale-orange powder.

### Example 18

Preparation of sepiolite adduct with oxidised serinol pyrrole 10 g of sepiolite are placed in a 250-ml flask equipped with a magnetic stirrer, and 15 ml of acetone are added. The mixture is sonicated for 10 minutes, then 0.5 g of oxidised serinol pyrrole, as in Example 15, is added as well as another 10 ml of acetone. The mixture is sonicated for another 30 minutes. At this point, the solvent is removed under reduced pressure and the mixture is stirred, using the magnetic stirrer, for 2 hours at a temperature of 150 °C. At the end of the reaction, said mixture is left to cool down to room temperature. The product is in the form of a sand-coloured powder.

### Example 19

Preparation of montmorillonite adduct with oxidised serinol pyrrole 5 g of montmorillonite are placed in a 250-ml flask equipped with a magnetic stirrer, and 15 ml of acetone are added. The mixture is sonicated for 10 minutes, then 0.5 g of oxidised serinol pyrrole, as in Example 15, is added as well as another 10 ml of acetone. The mixture is sonicated for another 30 minutes. At this point, the solvent is removed under reduced pressure and the mixture is stirred, using the magnetic stirrer, for 2 hours at a temperature of 150 °C. At the end of the reaction, said mixture is left to cool down to room temperature. The product is in the form of a light-brown powder.

### EXAMPLES 20-27 - Preparation of super-adducts formed of sp²-hybridised carbon allotropes and adducts consisting of inorganic oxide hydroxide with pyrrolic compounds

### Example 20

### Preparation formed from CB326 carbon black and silica/serinol pyrrole adduct. The silica/serinol pyrrole adduct was prepared as in Example 6.

The following are sequentially poured into a 250-ml flask equipped with a magnetic stirrer: 10 g of CB326 and 10 g of silica/SP adduct. The system is brought to a temperature of 180 °C for 3 hours using a reflux plant without a refrigerant therein. At the end of the reaction, said mixture is left to cool down to room temperature and the product that is primarily black in colour is retrieved.

### Example 21

### Preparation formed from CB326 carbon black and silica/serinol pyrrole adduct.

The silica/serinol pyrrole adduct was prepared as in Example 6.

The following are sequentially poured into a 250-ml flask equipped with a magnetic stirrer: 10 g of CB326 and 5 g of silica/SP adduct. The system is brought to a temperature of 180 °C for 3 hours using a reflux plant without a refrigerant therein. At the end of the reaction, said mixture is left to cool down to room temperature and the product that is primarily black in colour is retrieved.

### Example 22

### Preparation formed from CB326 carbon black and silica/serinol pyrrole adduct.

The silica/serinol pyrrole adduct was prepared as in Example 6.

The preparation is the same as that specified in Example 20, except for the fact that 2.5 g of silica/SP are used, and therefore 25 wt.% with respect to the CB326.

### Example 23

### Preparation formed from CB326 carbon black and silica/1-dodecyl-2.5-dimethyl-1H-pyrrole adduct

The silica/1-dodecyl-2.5-dimethyl-1H-pyrrole adduct was prepared as in Example 8.

The following are sequentially poured into a 250-ml flask equipped with a magnetic stirrer: 10 g of CB326 and an amount of silica/DDcP that is equal to 50 wt.% with respect to the CB326 (5 g). The system is brought to a temperature of 180 °C for 3 hours using a reflux plant without a refrigerant therein. At the end of the reaction, said mixture is left to cool down to room temperature and the product that is primarily black in colour is retrieved.

### Example 24

### Preparation formed from CB326 carbon black and silica/1-dodecyl-2.5-dimethyl-1H-pyrrole adduct

The silica/1-dodecyl-2.5-dimethyl-1H-pyrrole adduct was prepared as in Example 8.

The preparation is the same as that stated in Example 23, except for the fact that 2.5 g of silica/1-dodecyl-2.5-dimethyl-1H-pyrrole adduct are used, and therefore 25 wt.% with respect to the CB326.

### Example 25

Preparation formed from CB326 carbon black and silica/1.2.5-trimethyl-1H-pyrrole adduct The silica/1.2.5-trimethyl-1H-pyrrole adduct was prepared as in Example 13.

The following are sequentially poured into a 250-ml flask equipped with a magnetic stirrer: 10 g of CB326 and an amount of silica/TMP that is equal to 50 wt.% with respect to the CB326 (5 g). The system is brought to a temperature of 180 °C for 3 hours using a reflux plant without a refrigerant therein. At the end of the reaction, said mixture is left to cool down to room temperature and the product that is primarily black in colour is retrieved.

### Example 26

Preparation formed from carbon nanotubes (CNT) and silica/serinol pyrrole adduct

The nanotubes used are Nanocyl 7000 nanotubes.

The silica/serinol pyrrole adduct was prepared as in Example 6.

The following are sequentially poured into a 250-ml flask equipped with a magnetic stirrer: 2 g of CNT and 2 g of silica/serinol pyrrole adduct. The system is brought to a temperature of 180 °C for 3 hours using a reflux plant without a refrigerant therein. At the end of the reaction, said mixture is left to cool down to room temperature and the product that is black in colour is retrieved.

### Example 27

Preparation formed from graphite having a high surface area (HSAG) and a silica/serinol pyrrole adduct

The high surface area graphite (HSAG) used is Synthetic Graphite 8427, purchased from Asbury Graphite Mills Inc., which has a minimum carbon content equal to 99.8 wt.% and a surface area equal to 330 m²/g.

The silica/serinol pyrrole adduct was prepared as in Example 6.

The following are sequentially poured into a 250-ml flask equipped with a magnetic stirrer: 2 g of HSAG and 2 g of silica/serinol pyrrole adduct. The system is brought to a temperature of 180 °C for 3 hours using a reflux plant without a refrigerant therein. At the end of the reaction, said mixture is left to cool down to room temperature and the product that is black in colour is retrieved.

### Example 28

Dispersibility tests of the super-adduct
3 suspensions A, B and C were prepared as follows.

Suspension A (invention): 1 mg of super-adduct obtained as in Example 22 was mixed with 1 ml of distilled water.

Suspension B (invention): 1 mg of super-adduct obtained as in Example 27 was mixed with 1 ml of distilled water.

Suspension C (comparison): 1 mg of carbon black (CB N326) and silica were mixed with 1 ml of distilled water.

The suspensions A, B and C were then sonicated for 30 minutes, left to stand for 4 months, re-observed and photographed.

Fig. 1a, 1b and 1c show the photographs of suspensions A, B and C after the rest period of 4 months.

The observations at the end of the rest period have made it possible to prove how suspensions A and B are still homogeneously dispersed, while suspension C has completely separated.

### Example 29

### Thermogravimetric analysis (TGA) of the super-adduct

A first sample formed of carbon black (CB N326) and silica, prepared at 180 °C for 3 hours, and a second sample formed of a super-adduct that is obtained in accordance with Example 22 were subjected to thermogravimetric analysis (TGA).

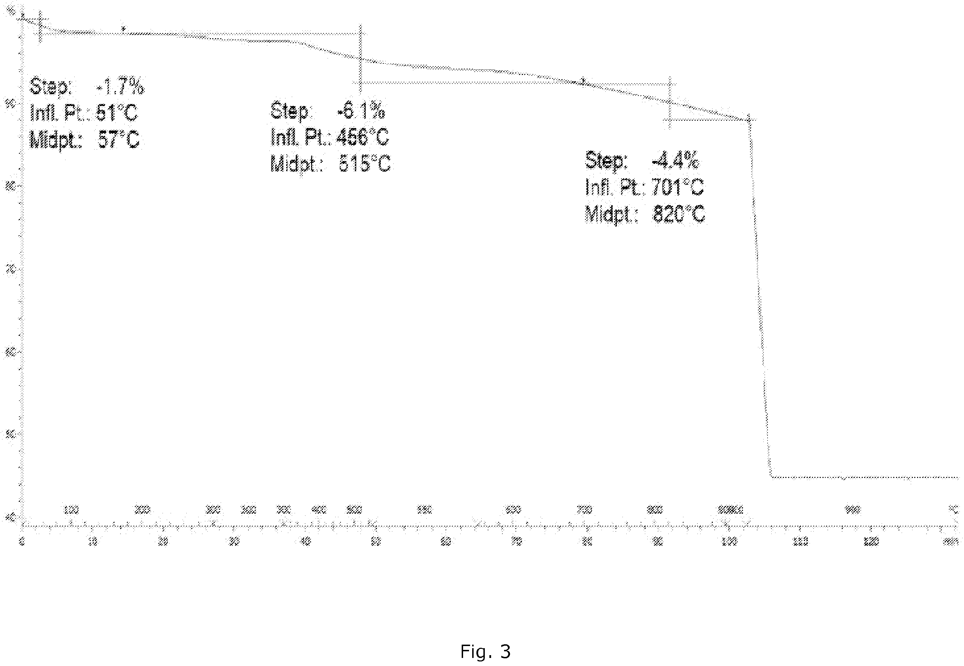

Fig. 2 and Fig. 3 respectively show the curves of the TGA of the first sample (comparison) and of the second sample (invention).

The analyses of the thermogravimetric curves show that both the samples exhibit the same loss of mass between 30 °C and 150 °C, which is associated with the loss in water that is physically adsorbed on the surface of the compounds.

Between 150 °C and 700 °C, the curve in Fig. 3 shows a loss of mass that is twice as large as that in the corresponding zone in Fig. 2. This loss of mass can be attributed to the loss of the pyrrole fraction in the super-adduct. Further analyses have confirmed functionalisation yields in the super-adduct that are also equal to 98 %.

This therefore shows that the super-adduct that comprises functionalising an sp²-hybridised carbon allotrope with the adduct is formed by means of a highly efficient atomic reaction, which does not release a significant amount of byproduct.

### Example 30

### Preparation of a compound for tyres comprising a super-adduct

### Materials

Natural rubber from *Hevea brasiliensis* (NR). Commercial name: SIR20 from Eatern GR Thailand - Chonburi. The Mooney viscosity (M_{L(1+4)} @ 100 °C) is equal to 73 Mooney Units (M_{U}). The super-adduct was obtained according to the procedures described in Example 22.

The silica used is Zeosil 1165, purchased from Solvay. The data sheet states: specific surface area = 140 - 180 m²/g; loss in mass in drying (2 hours @ 105 °C) <=8.0 %; soluble salts (such as Na₂SO₄) < = 2.0 %.

The carbon black is CB N326, purchased from Cabot. The data sheet states: BET surface area = 78 m²/g, external surface area = 76 m²/g, oil absorption number = 72 cm³/100 g, loss in mass in drying < = 1.0 %.

Furthermore, the following products were used: bis(3-triethoxysilylpropyl) tetrasulfide (TESPT), ZnO (zinc oxide), stearic acid (Sogis), 6PPD ((1,3-dimethyl butyl)-N'-phenyl-p-phenylenediamine from Crompton), S (sulfur, from Solfotecnica), TBBS (N-tert-butyl-2-benzothiazyl sulfenamide, from Flexsys).

### Formulations

Some elastomeric compound samples were prepared, respectively indicated as A, B and C, which have the formulations given in the following table, Table 1. The quantities of ingredients are expressed in phr.

**Table 1**

| **Ingredients** ^{a} | **Sample A** | **Sample B** | **Sample C** |
|---|---|---|---|
| Natural rubber SIR20 | 100.00 | 100.00 | 100.00 |
| Carbon Black N326 | 60.00 | 60.00 | 55.00 |
| TESPT | 0.00 | 0.50 | 0.00 |
| SiO₂ Zeosil 1165 | 5.00 | 5.00 | 0.00 |
| CB N326/SiO₂/SP^{b} | 0.00 | 0.00 | 10.50 |

| | | | |
|---|---|---|---|
| ^{a}Other ingredients: ZnO 4.0, stearic acid 2.0, sulfur 1.2, 6PPD 2.0, TBBS 1.7. ^{b} Quantity of SP on the silica: 5 % (weight) | | | |

### Preparation of the samples

The natural rubber is loaded into a Brabender^{®} internal mixer and masticated at 145 °C for 1 minute. At this point, CB N326 and, where provided, the Zeosil 1165 silica, the super-adduct and the TESPT silane are placed inside the chamber of the Brabender^{®}, which were mixed for a further 4 minutes at 145 °C. The composite obtained is discharged at 145 °C.

The composite obtained in this way is re-loaded into the chamber of the mixer at 50 °C and masticated for 1 minute. Stearic acid, zinc oxide and 6PPD are then added and mixed for 2 minutes at 50 °C. Lastly, TBBS and sulfur are added and mixed for another 2 minutes at 50 °C.

The composition obtained in this way is discharged at 50 °C.

### Example 31

### Tests carried out on the compound samples in Example 30.

Samples A, B and C prepared in Example 30 are subjected to a "strain sweep" test in order to verify the dynamic mechanical properties thereof by means of shear stress.

The "strain sweep" test involves the application of dynamic strain by means of shear stress at a constant frequency and temperature, thereby increasing the extent of deformation.

### The test was carried out using a Monsanto RPA 2000 rheometer.

Samples A, B and C were held in the rheometer at 50 °C for 90 seconds; the stress was then applied at 50 °C when the extent of deformation was between 0.1 % and 25 %, with a frequency of 1 Hz, thus increasing the extent of the deformation in the range indicated above. This treatment is carried out in order to override the "previous thermomechanical history".

The samples were then vulcanised at 170 °C for 20 minutes, at a frequency of 1.667 Hz and an angle of 6.98 % (0.5 rad). The vulcanised samples are left in the device for 10 minutes at 50 °C. The sinusoidal stress was then applied under the same conditions already stated, at 50 °C. The sinusoidal stress is then re-applied, again under the same experimental conditions.

Curves are then obtained that show the value for the moduli as a function of the extent of the deformation. These moduli are illustrated below. The modulus G' is the storage modulus, the modulus G" is the loss modulus. The ratio G"/G' is referred to as Tan Delta. The strain sweep test is used to obtain the values for the following parameters: G'_{γ=0.28%}, which is the G' value at minimum deformation, ΔG', which is the difference between the G' value at minimum deformation and the G' value measured at the maximum deformation reached, G"max, which is the maximum G" value observed in the G" curve, (Tan Delta)ₘₐₓ, which is the maximum Tan Delta value observed in the curve.

**Table 2. Main results of the strain sweep tests for the composites described in Table 1**

| **Sample** | **G' min (MPa)** | **G" max (MPa)** | **ΔG' (MPa)** | **ΔG'/G'min** |
|---|---|---|---|---|
| **Sample A** | 4.13 | 0.51 | 3.25 | 0.76 |
| **Sample B** | 3.27 | 0.42 | 1.94 | 0.59 |
| **Sample C** | 2.42 | 0.29 | 1.30 | 0.54 |

All of the samples display a Payne effect, as expected, with sample A demonstrating the greater Payne effect and sample C demonstrating the smaller Payne effect.

In the same way, the highest Tan δ values occurred in sample A, while the lowest Tan δ values were obtained by sample C.

The low Tan δ values make it possible to assert that sample C promotes a smaller amount of energy dissipation in a compound based on Nr and CB, as could be imagined for truck tyres, for example.

The analyses conducted show that the composites comprising the super-adduct CB N326/silica/SP display the best balance of properties: vulcanisation behaviour, Payne effect and hysteresis as well as tensile properties.

It therefore seems to be reasonable to believe that the positive results cited are a result of the presence of the super-adduct in the elastomeric composition.

## Claims

1. Adduct obtainable by interacting:
a) a pyrrolic compound of general formula (I) 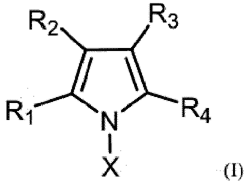
wherein R₁, R₂, R₃, R₄ are independently selected from the group consisting of: hydrogen, C₁-C₃ alkyl, linear or branched C₂-C₁₈ alkenyl or alkynyl, aryl, linear or branched C₁-C₁₈ alkyl-aryl, linear or branched C₂-C₁₈ alkenyl-aryl, linear or branched C₂-C₁₈ alkynyl-aryl and heteroaryl,
and X is selected from the group consisting of: 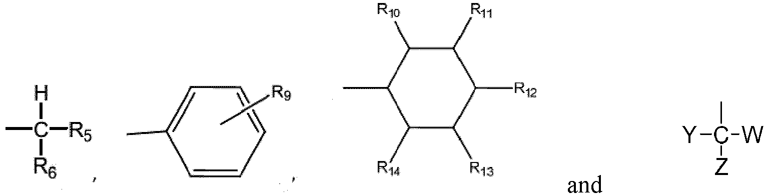
wherein R₅ and R₆ are independently selected from the group consisting of: hydrogen, C₁-C₁₈ alkyl, linear or branched C₂-C₁₈ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl and heteroaryl;
or either or both R5 or R6 is/are 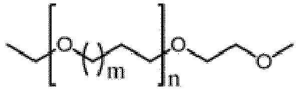 where m = 0, 1, 2 and n = 1-30
where, if only one of R₅ or R₆ is 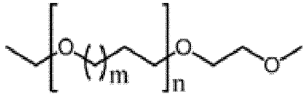 where m = 0, 1, 2 and n = 1-30, the other is selected from the group consisting of: hydrogen, C₁-C₁₈ alkyl and linear or branched C₂-C₁₈ alkenyl or alkynyl;
or R₅ and/or R₆ are: 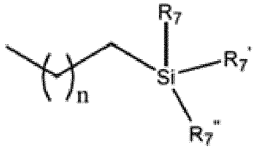 where n = 0, 1, 2, 3
and R₇, R_{7'}, R_{7"} are independently selected from the group consisting of:
C₁-C₄ alkyl; C₁-C₄ oxygen-alkyl;
or R₅ and/or R₆ are: 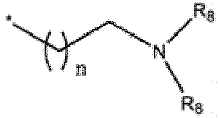 where n = 0, 1, 2, 3
and R₈ is selected from the group consisting of: C₁-C₄ alkyl;
or R₅ and/or R₆ are: 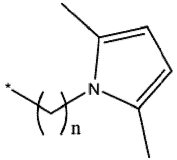
where n is an integer between 1 and 10;
or R₅ and/or R₆ are: 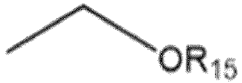
and R₁₅ is selected from the group consisting of: hydrogen, linear or branched C₁-C₂₂ alkyl, linear or branched C₂-C₂₂ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl, linear or branched C₂-C₂₂ acyl-alkyl, linear or branched C₃-C₂₂ acyl-alkenyl or acyl-alkynyl, acyl-aryl, acyl-alkyl-aryl comprising linear or branched C₂-C₂₂ acyl-alkyl, acyl-alkenyl-aryl comprising linear or branched C₃-C₂₂ acyl-alkenyl, acyl-alkynyl-aryl comprising linear or branched C₃-C₂₂ acyl-alkynyl and heteroaryl;
or R₅ and/or R₆ are: 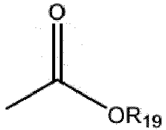
and R₁₉ is selected from the group consisting of: hydrogen, linear or branched C₁-C₂₂ alkyl, linear or branched C₂-C₂₂ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl and heteroaryl;
wherein R₉ is selected from the group consisting of: hydrogen, alkyl, aryl, benzyl, amine, alkylamine, arylamine, benzylamine and amine aryl;
wherein R₁₀, R₁₁, R₁₂, R₁₃ and R₁₄ are independently selected from the group consisting of: hydrogen, C₁-C₁₈ alkyl, linear or branched C₂-C₁₈ alkenyl or alkynyl and 1-(4-aminocyclohexyl) methylene; and
wherein Y, Z and W are independently selected from a first group consisting of: hydrogen, C₁-C₁₈ alkyl and linear or branched C₂-C₁₈ alkenyl or alkynyl, or from a second group consisting of: 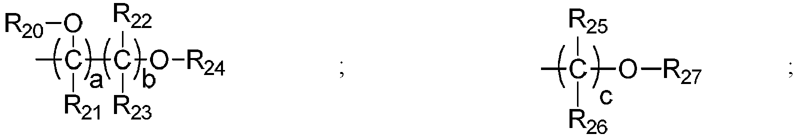 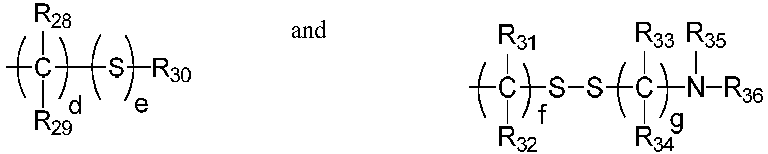
wherein R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆ are independently selected from the group consisting of: hydrogen, C₁-C₁₈ alkyl, linear or branched C₂-C₁₈ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl, heteroaryl and carboxyl;
where e is an integer between 1 and 4 and where a, b, c, d, f and g are, independently of one another, integers between 1 and 12;
wherein at least one of Y, W and Z is selected from said second group,
or an oxidized form of said pyrrolic compound of general formula (I) and
b. an inorganic oxide hydroxide, comprising an oxide and/or a hydroxide, and selected from the group consisting of silica, sheet silicates, mixed oxides consisting of aluminium and magnesium, alumina and silicoaluminates, wherein the ratio between the weight of said inorganic oxide hydroxide and said pyrrolic compound is greater than or equal to 10.

2. Adduct according to claim 1, wherein said ratio between the weight of said inorganic oxide hydroxide and said pyrrolic compound is greater than or equal to 50, more preferably greater than or equal to 100, and even more preferably greater than or equal to 500.

3. Adduct according to any one of the preceding claims, wherein
Y and Z are independently selected from said second group,
W is selected from said first group,
R₂₀, R₂₁, R₂₅, R₂₆, R₂₈, R₂₉, R₃₁ and R₃₂ are independently selected from the group consisting of: hydrogen, C₁-C₁₈ alkyl and linear or branched C₂-C₁₈ alkenyl or alkynyl;
and R₂₂, R₂₃, R₂₄, R₂₇, R₃₀, R₃₃, R₃₄, R₃₅ and R₃₆ are independently selected from the group consisting of: hydrogen, C₁-C₁₈ alkyl, linear or branched C₂-C₁₈ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl, heteroaryl and carboxyl.

4. Adduct according to any one of claims 1 and 2, wherein
Y is selected from said second group,
W and Z are independently selected from said first group,
R₂₀, R₂₁, R₂₅, R₂₆, R₂₈, R₂₉, R₃₁ and R₃₂ are independently selected from the group consisting of: hydrogen, C₁-C₁₈ alkyl and linear or branched C₂-C₁₈ alkenyl or alkynyl;
and R₂₂, R₂₃, R₂₄, R₂₇, R₃₀, R₃₃, R₃₄, R₃₅ and R₃₆ are independently selected from the group consisting of: hydrogen, C₁-C₁₈ alkyl, linear or branched C₂-C₁₈ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl, heteroaryl and carboxyl.

5. Adduct according to any one of the preceding claims, wherein said pyrrolic compound is serinol pyrrole.

6. Adduct according to any one of the preceding claims, wherein said inorganic oxide hydroxide is a sheet silicate selected from one of the groups consisting of serpentine-kaolin, talc-pyrophyllite, smectite, vermiculite, mica, chlorites, palygorskite, sepiolite, allophane, imogolite and hydrotalcite.

7. Method for preparing an adduct according to at least one of the preceding claims, comprising:
a. providing a first amount by weight of said pyrrolic compound,
b. providing a second amount by weight of an inorganic oxide hydroxide such that said second amount by weight is at least ten times greater than said first amount by weight of said pyrrolic compound, and
c. mixing said first amount by weight of said inorganic oxide hydroxide with said second amount by weight by supplying energy in order to form said adduct.

8. Method for preparing an adduct according to at least one of claims 1 to 6, comprising
a) mixing
- an amine composition of general formula (II) 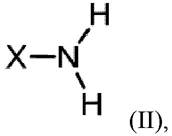
wherein X is selected from the group consisting of: 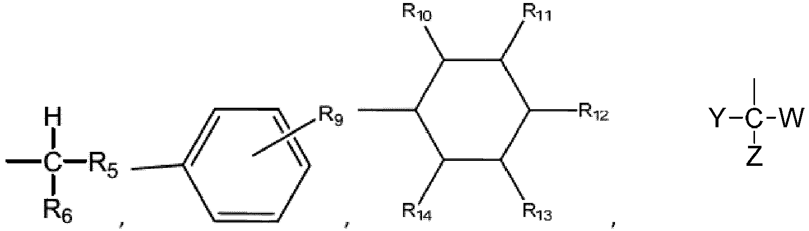 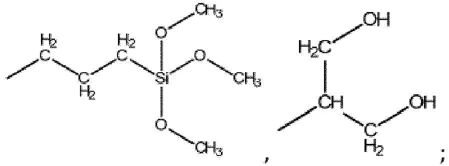
wherein
R₅ and R₆ are independently selected from the group consisting of: hydrogen, C₁-C₁₈ alkyl, linear or branched C₂-C₁₈ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl and heteroaryl;
or R₅ or R₆ are independently 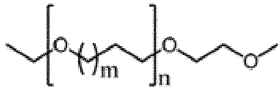 where m = 0, 1, 2 and n = 1-30,
where, if only one of R₅ or R₆ is 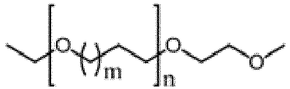 where m = 0, 1, 2 and n = 1-30,
then the other is selected from the group consisting of: hydrogen, C₁-C₁₈ alkyl and linear or branched C₂-C₁₈ alkenyl or alkynyl;
or R₅ or R₆ are 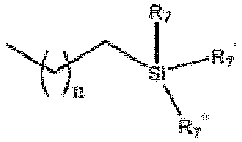 where n = 0, 1, 2, 3
and R₇, R_{7'}, R_{7"} are independently selected from the group consisting of: C₁-C₄ alkyl and C₁-C₄ oxygen-alkyl;
or R₅ and/or R₆ are: 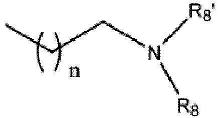
where n is an integer between 0 and 10 and R₈ and R_{8'} are independently selected from the group consisting of hydrogen and C₁-C₄ alkyl;
or R₅ and/or R₆ are: 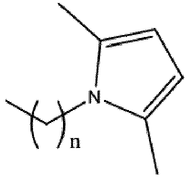
where n is an integer between 0 and 10,
or R₅ and/or R₆ are: 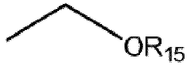
and R₁₅ is selected from the group consisting of: hydrogen, linear or branched C₁-C₂₂ alkyl, linear or branched C₂-C₂₂ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl, linear or branched C₂-C₂₂ acyl-alkyl, acyl-alkynyl or linear or branched C₃-C₂₂ acyl-alkenyl, acyl-aryl, acyl-alkyl-aryl comprising linear or branched C₂-C₂₂ acyl-alkyl, acyl-alkenyl-aryl comprising linear or branched C₃-C₂₂ acyl-alkenyl, acyl-alkynyl-aryl comprising linear or branched C₃-C₂₂ acyl-alkynyl and heteroaryl;
or R5 and/or R6 are: 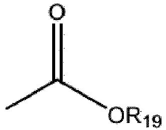
and R₁₉ is selected from the group consisting of: hydrogen, linear or branched C₁-C₂₂ alkyl, linear or branched C₂-C₂₂ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl and heteroaryl;
wherein R₉ is selected from the group consisting of: hydrogen, alkyl, aryl, benzyl, amine, alkylamine, arylamine, benzylamine and amine aryl;
wherein R₁₀, R₁₁, R₁₂, R₁₃ and R₁₄ are independently selected from the group consisting of: hydrogen, C₁-C₁₈ alkyl, linear or branched C₂-C₁₈ alkenyl or alkynyl, 1-(4-aminocyclohexyl) methylene;
and Y, Z and W are independently selected from a first group consisting of: hydrogen, C₁-C₁₈ alkyl and linear or branched C₂-C₁₈ alkenyl or alkynyl, or from a second group consisting of: 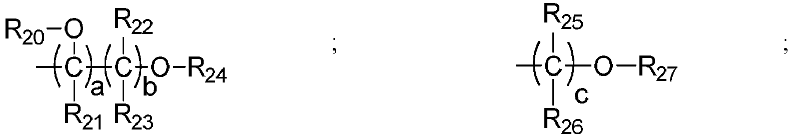 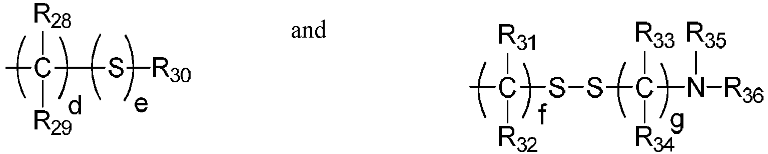
wherein R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅ and R₃₆ are independently selected from the group consisting of: hydrogen, C₁-C₁₈ alkyl, linear or branched C₂-C₁₈ alkenyl or alkynyl, aryl, linear or branched C₁-C₂₂ alkyl-aryl, linear or branched C₂-C₂₂ alkenyl-aryl, linear or branched C₂-C₂₂ alkynyl-aryl, heteroaryl and carboxyl,
where e is an integer between 1 and 4 and where a, b, c, d, f and g are, independently of one another, integers between 1 and 12;
wherein at least one of Y, W and Z is selected from said second group,
- with a carbonyl composition of general formula (III) 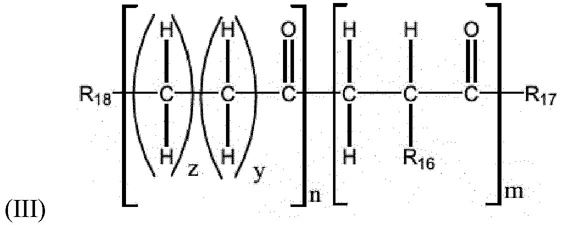
wherein n = 1-1000, m = 1-1000, y = 0-1, z = 0-1
wherein R₁₆ is selected from the group consisting of: hydrogen and methyl;
and R₁₇ and R₁₈ are selected from the group consisting of: hydrogen, alkyl or alkenyl, linear or branched C₂-C₃₀ alkynyl, aryl, C₂-C₃₀ alkyl-aryl, linear or branched C₂-C₃₀ alkenyl-aryl, C₂-C₃₀ alkynyl-aryl and heteroaryl;
- and with an inorganic oxide hydroxide such that the amount by weight of said inorganic oxide hydroxide is at least ten times greater than the sum of the amount by weight of said amine composition and said carbonyl composition, and
b) supplying energy in order to obtain said adduct.

9. Method according to either claim 7 or claim 8, wherein said pyrrolic compound is oxidized before being mixed with said inorganic oxide hydroxide.

10. Method according to claim 9, wherein said pyrrolic compound is oxidized by an oxygen-donor composition selected from the group consisting of H₂O₂, MnO₂, KMnO₄, HNO₃, NaClO (sodium hypochlorite), NaClO₂, H₂SO₄/HNO₃, NaIO₄, HIO₄, OsO₄, Pb(C₂H₃O₂)₄, HNO₃/HNO₂, CO₂, CO, dicumyl peroxide, RuO₂, RuO₄, SeO₂, O₃, periodinane, peracetic acid, iodosobenzoic acid, nicotinamide adenine dinucleotide (NAD+) and laccase or oxidase enzymes.

11. Method according to any one of claims 7 to 10, wherein said energy is supplied in the absence of solvents, preferably wherein the mixing and the supply of energy are carried out in the absence of solvents.

12. Method according to any one of claims 7 to 10, wherein the mixing and the supply of energy are carried out in the presence of at least one solvent, preferably selected from the group consisting of alcohols, aldehydes, ketones, esters, ethers and water, more preferably a water-based solvent.

13. Super-adduct, comprising
a. an adduct according to any one of claims 1 to 6, including said pyrrolic compound and said inorganic oxide hydroxide, and
b. an sp²-hybridized carbon allotrope or the derivatives thereof.

14. Method for producing a super-adduct according to claim 13, comprising
a. providing a third amount by weight of an adduct according to any one of claims 1 to 6, including said pyrrolic compound and said inorganic oxide hydroxide,
b. providing a fourth amount by weight of an sp²-hybridized carbon allotrope or the derivatives thereof, and
c. mixing said third amount by weight of said adduct with said fourth amount by weight of said sp²-hybridized carbon allotrope or the derivatives thereof by supplying energy, in order to obtain said super-adduct.

15. Elastomeric composition, comprising an elastomer and a filler, wherein said filler comprises a super-adduct according to claim 13.

## Patentansprüche

1. Addukt, das erhalten werden kann durch Zusammenwirken:
a) einer Pyrrol-Verbindung der allgemeinen Formel (I) 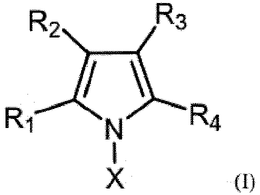
wobei R₁, R₂, R₃, R₄ unabhängig ausgewählt sind aus der Gruppe bestehend aus: Wasserstoff, C₁-C₃-Alkyl, linearem oder verzweigtem C₂-C₁₈-Alkenyl oder -Alkinyl, Aryl, linearem oder verzweigtem C₁-C₁₈-Alkyl-Aryl, linearem oder verzweigtem C₂-C₁₈-Alkenyl-Aryl, linearem oder verzweigtem C₂-C₁₈-Alkinyl-Aryl und Heteroaryl,
und X ausgewählt ist aus der Gruppe bestehend aus: 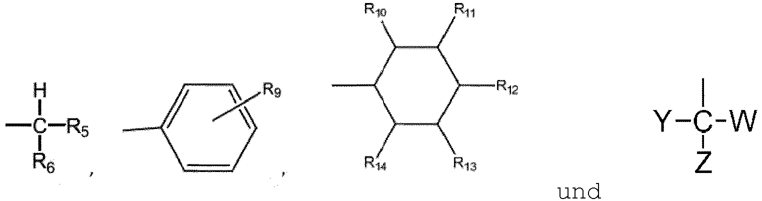
wobei R₅ und R₆ unabhängig ausgewählt sind aus der Gruppe bestehend aus: Wasserstoff, C₁-C₁₈-Alkyl, linearem oder verzweigtem C₂-C₁₈-Alkenyl oder -Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl und Heteroaryl;
oder einer oder beide von R₅ oder R₆ 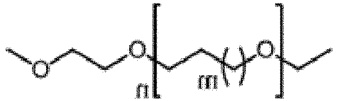 wobei m = 0, 1, 2 und n = 1 - 30
ist/sind, wobei, wenn nur eines von R₅ oder R₆ 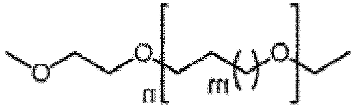 wobei m = 0, 1, 2 und n = 1 - 30,
ist, der andere ausgewählt ist aus der Gruppe bestehend aus: Wasserstoff, C₁-C₁₈-Alkyl und linearem oder verzweigtem C₂-C₁₈-Alkenyl oder -Alkinyl;
oder R₅ und/oder R₆: 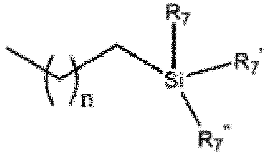 wobei n = 0, 1, 2, 3
sind, und R₇, R₇' , R₇" unabhängig ausgewählt sind aus der Gruppe bestehend aus:
C₁-C₄-Alkyl; C₁-C₄-Sauerstoff-Alkyl;
oder R₅ und/oder R₆ sind: 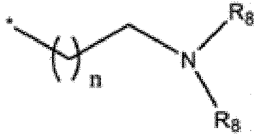 wobei n = 0, 1, 2, 3
und R₈ ausgewählt ist aus der Gruppe bestehend aus: C₁-C₄-Alkyl;
oder R₅ und/oder R₆ sind: 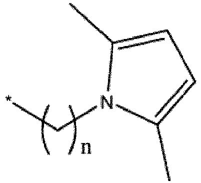
wobei n eine ganze Zahl zwischen 1 und 10 ist; oder R₅ und/oder R₆ sind: 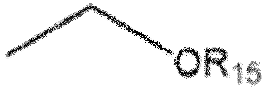
und R₁₅ ist ausgewählt aus der Gruppe bestehend aus: Wasserstoff, linearem oder verzweigtem C₁-C₂₂-Alkyl, linearem oder verzweigtem C₂-C₂₂-Alkenyl oder -Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl, linearem oder verzweigtem C₂-C₂₂-Acyl-Alkyl, linearem oder verzweigtem C₃-C₂₂-Acyl-Alkenyl oder -Acyl-Alkinyl, Acyl-Aryl, Acyl-Alkyl-Aryl umfassend lineares oder verzweigtes C₂-C₂₂-Acyl-Alkyl, Acyl-Alkenyl-Aryl umfassend lineares oder verzweigtes C₃-C₂₂-Acyl-Alkenyl, Acyl-Alkinyl-Aryl umfassend lineares oder verzweigtes C₃-C₂₂-Acyl-Alkinyl und Heteroaryl;
oder R₅ und/oder R₆ sind: 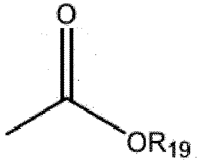
und R₁₉ ausgewählt ist aus der Gruppe bestehend aus: Wasserstoff, linearem oder verzweigtem C₁-C₂₂-Alkyl, linearem oder verzweigtem C₂-C₂₂-Alkenyl oder -Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl und Heteroaryl;
wobei R₉ ausgewählt ist aus der Gruppe bestehend aus: Wasserstoff, Alkyl, Aryl, Benzyl, Amin, Alkylamin, Arylamin, Benzylamin und Amin-Aryl;
wobei R₁₀, R₁₁, R₁₂, R₁₃ und R₁₄ unabhängig ausgewählt sind aus der Gruppe bestehend aus:
Wasserstoff, C₁-C₁₈-Alkyl, linearem oder verzweigtem C₂-C₁₈-Alkenyl oder -Alkinyl und 1-(4-Aminocyclohexyl)methylen; und
wobei Y, Z und W unabhängig ausgewählt sind aus einer ersten Gruppe bestehend aus: Wasserstoff, C₁-C₁₈-Alkyl und linearem oder verzweigtem C₂-C₁₈-Alkenyl oder -Alkinyl; oder aus einer zweiten Gruppe bestehend aus: 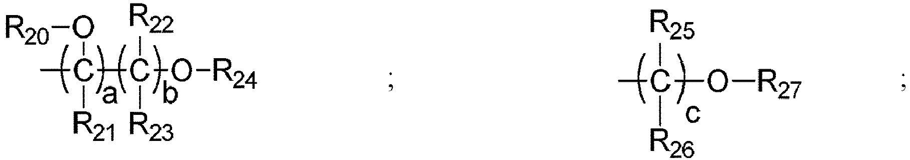 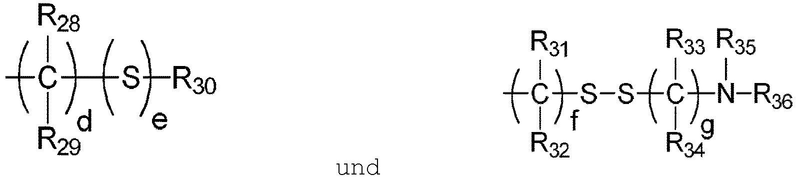
wobei R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆ unabhängig ausgewählt sind aus der Gruppe bestehend aus: Wasserstoff, C₁-C₁₈-Alkyl, linearem oder verzweigtem C₂-C₁₈-Alkenyl oder -Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl, Heteroaryl und Carboxyl;
wobei e eine ganze Zahl zwischen 1 und 4 ist und wobei a, b, c, d, f und g unabhängig voneinander ganze Zahlen zwischen 1 und 12 sind;
wobei zumindest eines von Y, W und Z aus der zweiten Gruppe ausgewählt ist,
oder einer oxidierten Form der Pyrrol-Verbindung der allgemeinen Formel (I)
und
b. eines anorganischen Oxid-Hydroxids, umfassend ein Oxid und/oder ein Hydroxid, und ausgewählt aus der Gruppe bestehend aus Silica, Phyllosilikate, Mischoxide bestehend aus Aluminium und Magnesium, Aluminiumoxid und Silikoaluminaten, wobei das Verhältnis zwischen dem Gewicht des anorganischen Oxid-Hydroxids und der Pyrrol-Verbindung größer als oder gleich 10 ist.

2. Addukt nach Anspruch 1, wobei das Verhältnis zwischen dem Gewicht des anorganischen Oxid-Hydroxids und der Pyrrol-Verbindung größer als oder gleich 50 ist, noch bevorzugter größer als oder gleich 100, und sogar noch bevorzugter größer als oder gleich 500.

3. Addukt nach einem der vorhergehenden Ansprüche, wobei Y und Z unabhängig aus der zweiten Gruppe ausgewählt sind,
W aus der ersten Gruppe ausgewählt ist,
R₂₀, R₂₁, R₂₅, R₂₆, R₂₈, R₂₉, R₃₁ und R₃₂ unabhängig ausgewählt sind aus der Gruppe bestehend aus: Wasserstoff, C₁-C₁₈-Alkyl und linearem oder verzweigtem C₂-C₁₈-Alkenyl oder -Alkinyl,
und R₂₂, R₂₃, R₂₄, R₂₇, R₃₀, R₃₃, R₃₄, R₃₅ und R₃₆ unabhängig ausgewählt sind aus der Gruppe bestehend aus: Wasserstoff, C₁-C₁₈-Alkyl, linearem oder verzweigtem C₂-C₁₈-Alkenyl oder - Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl, Heteroaryl und Carboxyl.

4. Addukt nach einem der Ansprüche 1 und 2, wobei Y aus der zweiten Gruppe ausgewählt ist,
W und Z unabhängig aus der ersten Gruppe ausgewählt sind, R₂₀, R₂₁, R₂₅, R₂₆, R₂₈, R₂₉, R₃₁ und R₃₂ unabhängig ausgewählt sind aus der Gruppe bestehend aus: Wasserstoff, C₁-C₁₈-Alkyl und linearem oder verzweigtem C₂-C₁₈-Alkenyl oder -Alkinyl,
und R₂₂, R₂₃, R₂₄, R₂₇, R₃₀, R₃₃, R₃₄, R₃₅ und R₃₆ unabhängig ausgewählt sind aus der Gruppe bestehend aus: Wasserstoff, C₁-C₁₈-Alkyl, linearem oder verzweigtem C₂-C₁₈-Alkenyl oder - Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl, Heteroaryl und Carboxyl.

5. Addukt nach einem der vorhergehenden Ansprüche, wobei die Pyrrol-Verbindung Serinol-Pyrrol ist.

6. Addukt nach einem der vorhergehenden Ansprüche, wobei das anorganische Oxid-Hydroxid ein Phyllosilikat ausgewählt aus einer der Gruppen bestehend aus Serpentin-Kaolin, Talk-Pyrophyllit, Smektit, Vermiculit, Mica, Chloriten, Palygorskit, Sepiolith, Allophan, Imogolit und Hydrotalcit.

7. Verfahren zur Herstellung eines Addukts nach mindestens einem der vorhergehenden Ansprüche,
wobei das Verfahren umfasst:
a. Bereitstellen einer ersten Menge nach Gewicht der Pyrrol-Verbindung,
b. Bereitstellen einer zweiten Menge nach Gewicht eines anorganischen Oxid-Hydroxids, so dass die zweite Menge nach Gewicht zumindest zehnmal größer ist als die erste Menge nach Gewicht der Pyrrol-Verbindung, und
c. Mischen der ersten Menge nach Gewicht des anorganischen Oxid-Hydroxids mit der zweiten Menge nach Gewicht durch Zuführen von Energie, um das Addukt zu bilden.

8. Verfahren zur Herstellung eines Addukts nach mindestens einem der Ansprüche 1 bis 6, umfassend
a) Mischen
- einer Amin-Zusammensetzung der allgemeinen Formel (II) 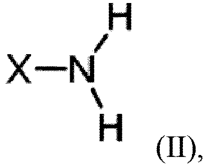
wobei X ausgewählt ist aus der Gruppe bestehend aus: 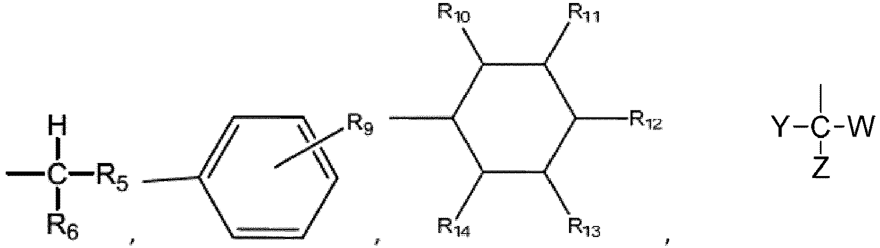 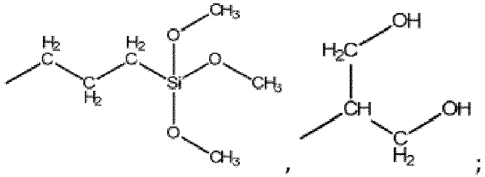
wobei
R₅ und R₆ unabhängig ausgewählt sind aus der Gruppe bestehend aus Wasserstoff, C₁-C₁₈-Alkyl, linearem oder verzweigtem C₂-C₁₈-Alkenyl oder -Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl und Heteroaryl;
oder R₅ oder R₆ sind unabhängig 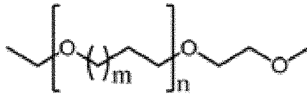 wobei m = 0, 1, 2 und n = 1 - 30,
sind, wobei, wenn nur eines von R₅ oder R₆ 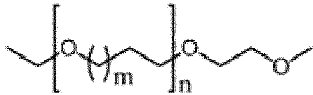 wobei m = 0, 1, 2 und n = 1 - 30,
ist, das jeweils andere ausgewählt ist aus der Gruppe bestehend aus: Wasserstoff, C₁-C₁₈-Alkyl und linearem oder verzweigtem C₂-C₁₈-Alkenyl oder -Alkinyl;
oder R₅ oder R₆ sind 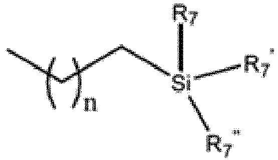 wobei n = 0, 1, 2, 3
sind und R₇, R₇' , R₇" unabhängig ausgewählt sind aus der Gruppe bestehend aus: C₁-C₄-Alkyl und C₁-C₄-Sauerstoff-Alkyl;
oder R₅ und/oder R₆: 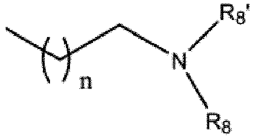
sind, wobei n eine ganze Zahl zwischen 0 und 10 ist und R₈ und R₈' unabhängig ausgewählt sind aus der Gruppe bestehend aus Wasserstoff und C₁-C₄-Alkyl;
oder R₅ und/oder R₆: 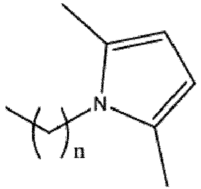
sind, wobei n eine ganze Zahl zwischen 0 und 10 ist, oder R₅ und/oder R₆: 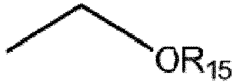
sind und R₁₅ ausgewählt ist aus der Gruppe bestehend aus: Wasserstoff, linearem oder verzweigtem C₁-C₂₂-Alkyl, linearem oder verzweigtem C₂-C₂₂-Alkenyl oder -Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl, linearem oder verzweigtem C₂-C₂₂-Acyl-Alkyl, Acyl-Alkinyl oder linearem oder verzweigtem C₃-C₂₂-Acyl-Alkenyl, Acyl-Aryl, Acyl-Alkyl-Aryl umfassend lineares oder verzweigtes C₂-C₂₂-Acyl-Alkyl, Acyl-Alkenyl-Aryl umfassend lineares oder verzweigtes C₃-C₂₂-Acyl-Alkenyl, Acyl-Alkinyl-Aryl umfassend lineares oder verzweigtes C₃-C₂₂-Acyl-Alkinyl und Heteroaryl;
oder R₅ und/oder R₆: 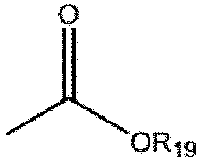
sind und R₁₉ ausgewählt ist aus der Gruppe bestehend aus: Wasserstoff, linearem oder verzweigtem C₁-C₂₂-Alkyl, linearem oder verzweigtem C₂-C₂₂-Alkenyl oder -Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl und Heteroaryl;
wobei R₉ ausgewählt ist aus der Gruppe bestehend aus: Wasserstoff, Alkyl, Aryl, Benzyl, Amin, Alkylamin, Arylamin, Benzylamin und Amin-Aryl;
wobei R₁₀, R₁₁, R₁₂, R₁₃ und R₁₄ unabhängig ausgewählt sind aus der Gruppe bestehend aus:
Wasserstoff, C₁-C₁₈-Alkyl, linearem oder verzweigtem C₂-C₁₈-Alkenyl oder -Alkinyl, 1-(4-Aminocyclohexyl)methylen;
und Y, Z und W unabhängig ausgewählt sind aus einer ersten Gruppe bestehend aus: Wasserstoff, C₁-C₁₈-Alkyl und linearem oder verzweigtem C₂-C₁₈-Alkenyl oder -Alkinyl; oder aus einer zweiten Gruppe bestehend aus: 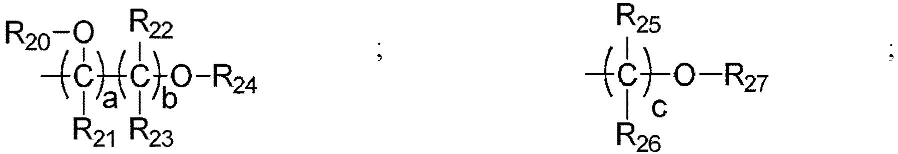 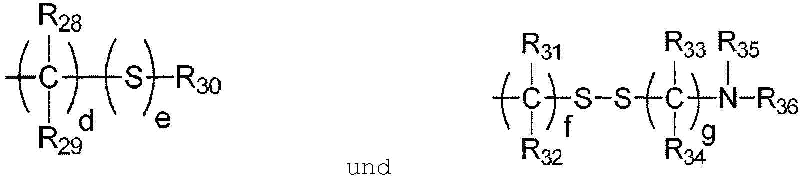
wobei R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅ und R₃₆ unabhängig ausgewählt sind aus der Gruppe bestehend aus: Wasserstoff, C₁-C₁₈-Alkyl, linearem oder verzweigtem C₂-C₁₈-Alkenyl oder -Alkinyl, Aryl, linearem oder verzweigtem C₁-C₂₂-Alkyl-Aryl, linearem oder verzweigtem C₂-C₂₂-Alkenyl-Aryl, linearem oder verzweigtem C₂-C₂₂-Alkinyl-Aryl, Heteroaryl und Carboxyl,
wobei e eine ganze Zahl zwischen 1 und 4 ist und wobei a, b, c, d, f und g unabhängig voneinander ganze Zahlen zwischen 1 und 12 sind;
wobei zumindest eines von Y, W und Z aus der zweiten Gruppe ausgewählt ist,
- mit einer Carbonyl-Zusammensetzung der allgemeinen Formel (III) 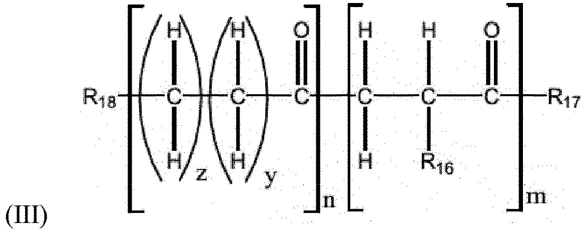
wobei n = 1 - 1000, m = 1 - 1000, y = 0 - 1, z = 0 - 1
wobei R₁₆ ausgewählt ist aus der Gruppe bestehend aus: Wasserstoff und Methyl;
und R₁₇ und R₁₈ ausgewählt sind aus der Gruppe bestehend aus: Wasserstoff, Alkyl oder Alkenyl, linearem oder verzweigtem C₂-C₃₀ Alkinyl, Aryl, C₂-C₃₀-Alkyl-Aryl, linearem oder verzweigtem C₂-C₃₀-Alkenyl-Aryl, C₂-C₃₀-Alkinyl-Aryl und Heteroaryl;
- und mit einem anorganischen Oxid-Hydroxid, so dass die Menge nach Gewicht des anorganischen Oxid-Hydroxids zumindest zehnmal größer ist als die Summe der Menge nach Gewicht der Amin-Zusammensetzung und der Carbonyl-Zusammensetzung, und b) Zuführen von Energie, um das Addukt zu erhalten.

9. Verfahren entweder nach Anspruch 7 oder nach Anspruch 8, wobei die Pyrrol-Verbindung oxidiert wird, bevor sie mit dem anorganischen Oxid-Hydroxid gemischt wird.

10. Verfahren nach Anspruch 9, wobei die Pyrrol-Verbindung durch eine Sauerstoffdonor-Zusammensetzung oxidiert wird, die aus der Gruppe ausgewählt ist, die aus H₂O₂, MnO₂, KMnO₄, HNO₃, NaClO (Natriumhypochlorit), NaClO₂, H₂SO₄/HNO₃, NaIO4, HIO₄, OsO4, Pb (C₂H₃O₂)₄, HNO₃/HNO₂, CO₂, CO, DicumylPeroxid, RuO₂, RuO₄, SeO₂, O₃, Periodinan, Peroxyessigsäure, Iodbenzoesäure, Nicotinamid-Adenin-Dinucleotid (NAD+) und Laccase- oder Oxidase-Enzymen besteht.

11. Verfahren nach einem der Ansprüche 7 bis 10, wobei die Energie in Abwesenheit von Lösungsmittel zugeführt wird, wobei vorzugsweise das Mischen und die Zufuhr von Energie in Abwesenheit von Lösungsmittel durchgeführt werden.

12. Verfahren nach einem der Ansprüche 7 bis 10, wobei das Mischen und die Zufuhr von Energie in Anwesenheit von zumindest einem Lösungsmittel durchgeführt werden, vorzugsweise ausgewählt aus der Gruppe bestehend aus Alkoholen, Aldehyden, Ketonen, Estern, Ethern und Wasser, noch bevorzugter einem auf Wasser basierenden Lösungsmittel.

13. Super-Addukt, umfassend
a. ein Addukt nach einem der Ansprüche 1 bis 6, das die Pyrrol-Verbindung und das anorganische Oxid-Hydroxid beinhaltet, und
b. ein sp²-hybridisiertes Kohlenstoff-Allotrop oder die Derivate davon.

14. Verfahren zur Herstellung eines Super-Addukts nach Anspruch 13, umfassend
a. Bereitstellen einer dritten Menge nach Gewicht eines Addukts nach einem der Ansprüche 1 bis 6, das die Pyrrol-Verbindung und das anorganische Oxid-Hydroxid beinhaltet,
b. Bereitstellen einer vierten Menge nach Gewicht des sp²-hybridisierten Kohlenstoff-Allotrops oder der Derivate davon, und
c. Mischen der dritten Menge nach Gewicht des Addukts mit der vierten Menge nach Gewicht des sp²-hybridisierten Kohlenstoff-Allotrops oder der Derivate davon durch Zuführen von Energie, um das Super-Addukt zu erhalten.

15. Elastomere Zusammensetzung, umfassend ein Elastomer und einen Füllstoff, wobei der Füllstoff ein Super-Addukt nach Anspruch 13 umfasst.

## Revendications

1. Adduit pouvant être obtenu par interaction :
a) d'un composé Pyrrolique de formule générale (I) 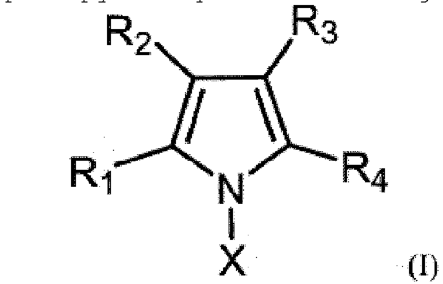
où R₁, R₂, R₃, R₄ sont indépendamment choisis dans le groupe constitué par : un hydrogène, un alkyle en C₁ à C₃, un alcényle ou alcynyle en C₂ à C₁₈ linéaire ou ramifié, un aryle, un alkyl-aryle en C₁ à C₁₈ linéaire ou ramifié, un alcényl-aryle en C₂ à C₁₈ linéaire ou ramifié, un alcynyl-aryle en C₂ à C₁₈ linéaire ou ramifié et un hétéroaryle,
et X est choisi dans le groupe constitué par : 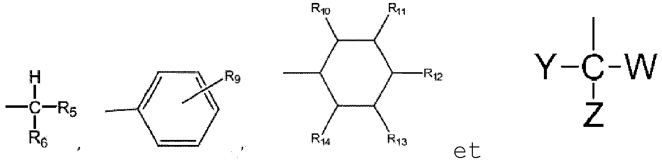
où R₅ et R₆ sont indépendamment choisis dans le groupe constitué par : un hydrogène, un alkyle en C₁ à C₁₈, un alcényle ou alcynyle en C₂ à C₁₈ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié et un hétéroaryle ;
ou R5 et/ou R6 représente/représentent 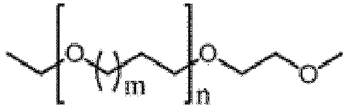 où m = 0, 1, 2 et n = 1 à 30, où, si un seul parmi R₅ et R₆ représente 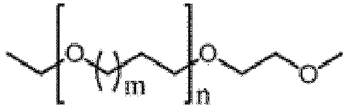 où m = 0, 1, 2 et n = 1 à 30,
l'autre est choisi dans le groupe constitué par : un hydrogène, un alkyle en C₁ à C₁₈ et un alcényle ou alcynyle en C₂ à C₁₈ linéaire ou ramifié ;
ou R₅ et/ou R₆ représente/représentent : 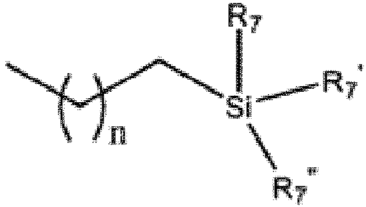 où n = 0, 1, 2, 3
et R₇, R_{7'}, R_{7"} sont indépendamment choisis dans le groupe constitué par : un alkyle en C₁ à C₄; un oxygène-alkyle en C₁ à C₄;
ou R₅ et/ou R₆ représente/représentent : 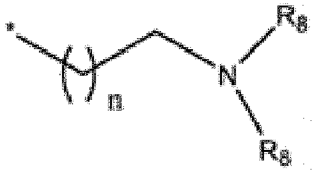 où n = 0, 1, 2, 3
et R₈ est choisi dans le groupe constitué par : un alkyle en C₁ à C₄ ;
ou R₅ et/ou R₆ représente/représentent : 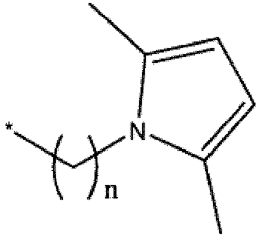
où n est un nombre entier compris entre 1 et 10 ; ou R₅ et/ou R₆ représente/représentent : 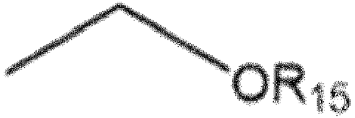
et R₁₅ est choisi dans le groupe constitué par : un hydrogène, un alkyle en C₁ à C₂₂ linéaire ou ramifié, un alcényle ou alcynyle en C₂ à C₂₂ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié, un acyl-alkyle en C₂ à C₂₂ linéaire ou ramifié, un acyl-alcényle ou acyl-alcynyle en C₃ à C₂₂ linéaire ou ramifié, un acyl-aryle, un acyl-alkyl-aryle comprenant un acyl-alkyle en C₂ à C₂₂ linéaire ou ramifié, un acyl-alcényl-aryle comprenant un acyl-alcényle en C₃ à C₂₂ linéaire ou ramifié, un acyl-alcynyl-aryle comprenant un acyl-alcynyle en C₃ à C₂₂ linéaire ou ramifié et un hétéroaryle ;
ou R₅ et/ou R₆ représente/représentent : 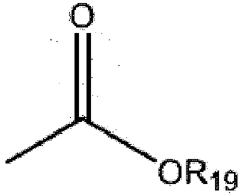
et R₁₉ est choisi dans le groupe constitué par : un hydrogène, un alkyle en C₁ à C₂₂ linéaire ou ramifié, un alcényle ou alcynyle en C₂ à C₂₂ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié et un hétéroaryle ;
où R₉ est choisi dans le groupe constitué par : un hydrogène, un alkyle, un aryle, un benzyle, une amine, une alkylamine, une arylamine, une benzylamine et une amine-aryle ;
où R₁₀, R₁₁, R₁₂, R₁₃ et R₁₄ sont indépendamment choisis dans le groupe constitué par : un hydrogène, un alkyle en C₁ à C₁₈, un alcényle ou alcynyle en C₂ à C₁₈ linéaire ou ramifié et le 1-(4-aminocyclohexyl)méthylène ; et
où Y, Z et W sont indépendamment choisis dans un premier groupe constitué par : un hydrogène, un alkyle en C₁ à C₁₈ et un alcényle ou alcynyle en C₂ à C₁₈ linéaire ou ramifié, ou dans un deuxième groupe constitué par : 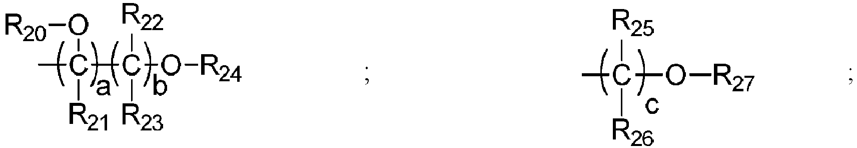 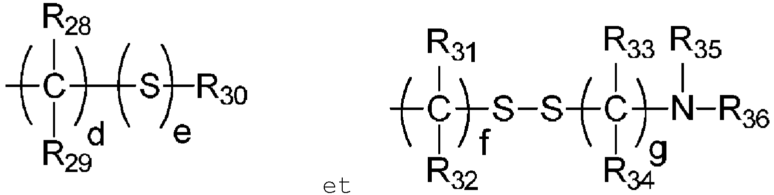
où R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆ sont indépendamment choisis dans le groupe constitué par : un hydrogène, un alkyle en C₁ à C₁₈, un alcényle ou alcynyle en C₂ à C₁₈ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié, un hétéroaryle et un carboxyle ;
où e est un nombre entier compris entre 1 et 4 et où a, b, c, d, f et g sont, indépendamment les uns des autres, des nombres entiers compris entre 1 et 12 ;
où au moins l'un parmi Y, W et Z est choisi dans ledit deuxième groupe,
ou une forme oxydée dudit composé pyrrolique de formule générale (I) et
b. d'un oxyde-hydroxyde inorganique, comprenant un oxyde et/ou un hydroxyde, et choisi dans le groupe constitué par la silice, des silicates en feuille, des oxydes mixtes constitués d'aluminium et de magnésium, l'alumine et les silicoaluminates, où le rapport entre le poids dudit oxyde-hydroxyde inorganique et dudit composé pyrrolique est supérieur ou égal à 10.

2. Adduit selon la revendication 1, dans lequel ledit rapport entre le poids dudit oxyde-hydroxyde inorganique et dudit composé pyrrolique est supérieur ou égal à 50, plus préférablement supérieur ou égal à 100, et encore plus préférablement supérieur ou égal à 500.

3. Adduit selon l'une quelconque des revendications précédentes, dans lequel
Y et Z sont indépendamment choisis dans ledit deuxième groupe,
W est choisi dans ledit premier groupe, R₂₀, R₂₁, R₂₅, R₂₆, R₂₈, R₂₉, R₃₁ et R₃₂ sont indépendamment choisis dans le groupe constitué par : un hydrogène, un alkyle en C₁ à C₁₈ et un alcényle ou alcynyle en C₂ à C₁₈ linéaire ou ramifié ;
et R₂₂, R₂₃, R₂₄, R₂₇, R₃₀, R₃₃, R₃₄, R₃₅ et R₃₆ sont indépendamment choisis dans le groupe constitué par : un hydrogène, un alkyle en C₁ à C₁₈, un alcényle ou alcynyle en C₂ à C₁₈ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié, un hétéroaryle et un carboxyle.

4. Adduit selon l'une quelconque des revendications 1 et 2, dans lequel
Y est choisi dans ledit deuxième groupe,
W et Z sont indépendamment choisis dans ledit premier groupe,
R₂₀, R₂₁, R₂₅, R₂₆, R₂₈, R₂₉, R₃₁ et R₃₂ sont indépendamment choisis dans le groupe constitué par : un hydrogène, un alkyle en C₁ à C₁₈ et un alcényle ou alcynyle en C₂ à C₁₈ linéaire ou ramifié ;
et R₂₂, R₂₃, R₂₄, R₂₇, R₃₀, R₃₃, R₃₄, R₃₅ et R₃₆ sont indépendamment choisis dans le groupe constitué par : un hydrogène, un alkyle en C₁ à C₁₈, un alcényle ou alcynyle en C₂ à C₁₈ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié, un hétéroaryle et un carboxyle.

5. Adduit selon l'une quelconque des revendications précédentes, dans lequel ledit composé pyrrolique est le sérinol-pyrrole.

6. Adduit selon l'une quelconque des revendications précédentes, dans lequel ledit oxyde-hydroxyde inorganique est un phyllosilicate choisi dans l'un des groupes constitués par la serpentine-kaolin, le talc-pyrophyllite, la smectite, la vermiculite, le mica, les chlorites, la palygorskite, la sépiolite, l'allophane, l'imogolite et l'hydrotalcite.

7. Procédé de préparation d'un adduit selon au moins l'une des revendications précédentes, comprenant le fait de :
a. fournir une première quantité en poids dudit composé pyrrolique,
b. fournir une deuxième quantité en poids d'un oxyde-hydroxyde inorganique de sorte que ladite deuxième quantité en poids soit au moins dix fois supérieure à ladite première quantité en poids dudit composé pyrrolique, et
c. mélanger ladite première quantité en poids dudit oxyde-hydroxyde inorganique avec ladite deuxième quantité en poids en apportant de l'énergie afin de former ledit adduit.

8. Procédé de préparation d'un adduit selon au moins l'une des revendications 1 à 6, comprenant le fait de
a) mélanger
- une composition d'amine de formule générale (II) 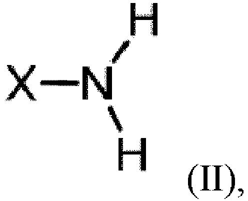
où X est choisi dans le groupe constitué par : 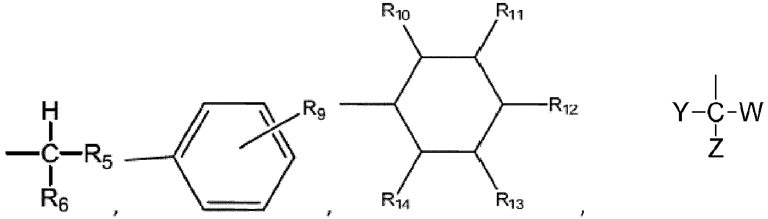 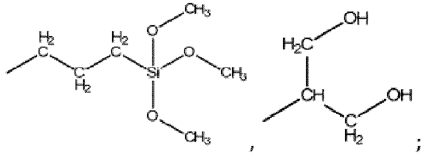
où
R₅ et R₆ sont indépendamment choisis dans le groupe constitué par : un hydrogène, un alkyle en C₁ à C₁₈, un alcényle ou alcynyle en C₂ à C₁₈ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié et un hétéroaryle ;
ou R₅ ou R₆ représente indépendamment 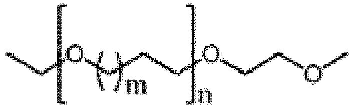 où m = 0, 1, 2 et n = 1 à 30,
où si un seul parmi R₅ et R₆ représente 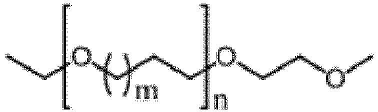 où m = 0, 1, 2 et n = 1 à 30,
alors l'autre est choisi dans le groupe constitué par : un hydrogène, un alkyle en C₁ à C₁₈ et un alcényle ou alcynyle en C₂ à C₁₈ linéaire ou ramifié ;
ou R₅ ou R₆ représente : 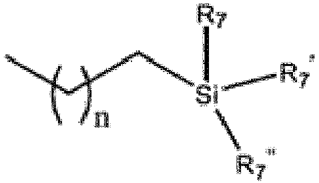 où n = 0, 1, 2, 3
et R₇, R_{7'}, R_{7"} sont indépendamment choisis dans le groupe constitué par : un alkyle en C₁ à C₄ et un oxygène-alkyle en C₁ à C₄ ;
ou R₅ et/ou R₆ représente/représentent : 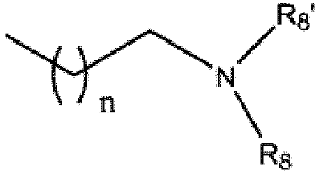
où n est un nombre entier compris entre 0 et 10 et R₈ et R_{8'} sont indépendamment choisis dans le groupe constitué par un hydrogène et un alkyle en C₁ à C₄ ;
ou R₅ et/ou R₆ représente/représentent : 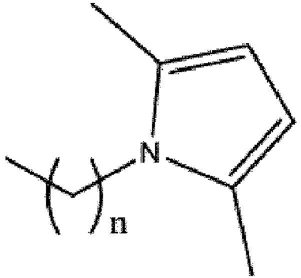
où n est un nombre entier compris entre 0 et 10, ou R₅ et/ou R₆ représente/représentent : 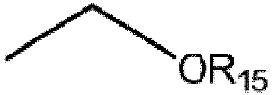
et R₁₅ est choisi dans le groupe constitué par : un hydrogène, un alkyle en C₁ à C₂₂ linéaire ou ramifié, un alcényle ou alcynyle en C₂ à C₂₂ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié, un acyl-alkyle en C₂ à C₂₂ linéaire ou ramifié, un acyl-alcynyle ou un acyl-alcényle en C₃ à C₂₂ linéaire ou ramifié, un acyl-aryle, un acyl-alkyl-aryle comprenant un acyl-alkyle en C₂ à C₂₂ linéaire ou ramifié, un acyl-alcényl-aryle comprenant un acyl-alcényle en C₃ à C₂₂ linéaire ou ramifié, un acyl-alcynyl-aryle comprenant un acyl-alcynyle en C₃ à C₂₂ linéaire ou ramifié et un hétéroaryle ;
ou R5 et/ou R6 représente/représentent : 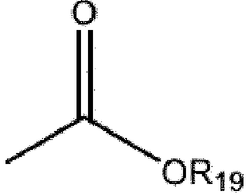
et R₁₉ est choisi dans le groupe constitué par : un hydrogène, un alkyle en C₁ à C₂₂ linéaire ou ramifié, un alcényle ou alcynyle en C₂ à C₂₂ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié et un hétéroaryle ;
où R₉ est choisi dans le groupe constitué par : un hydrogène, un alkyle, un aryle, un benzyle, une amine, une alkylamine, une arylamine, une benzylamine et une amine-aryle ;
où R₁₀, R₁₁, R₁₂, R₁₃ et R₁₄ sont indépendamment choisis dans le groupe constitué par : un hydrogène, un alkyle en C₁ à C₁₈, un alcényle ou alcynyle en C₂ à C₁₈ linéaire ou ramifié, le 1-(4-aminocyclohexyl)méthylène ;
et Y, Z et W sont indépendamment choisis dans un premier groupe constitué par : un hydrogène, un alkyle en C₁ à C₁₈ et un alcényle ou alcynyle en C₂ à C₁₈ linéaire ou ramifié, ou dans un deuxième groupe constitué par : 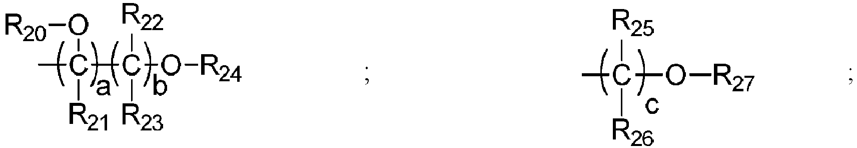 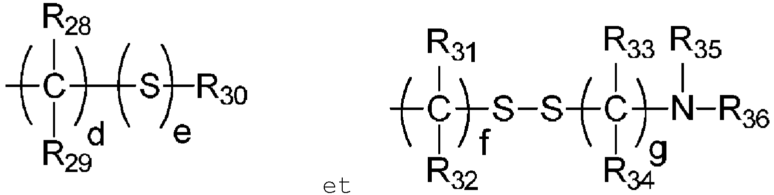
où R₂₀, R₂₁, R₂₂, R₂₃, R₂₄, R₂₅, R₂₆, R₂₇, R₂₈, R₂₉, R₃₀, R₃₁, R₃₂, R₃₃, R₃₄, R₃₅, R₃₆ sont indépendamment choisis dans le groupe constitué par : un hydrogène, un alkyle en C₁ à C₁₈, un alcényle ou alcynyle en C₂ à C₁₈ linéaire ou ramifié, un aryle, un alkyl-aryle en C₁ à C₂₂ linéaire ou ramifié, un alcényl-aryle en C₂ à C₂₂ linéaire ou ramifié, un alcynyl-aryle en C₂ à C₂₂ linéaire ou ramifié, un hétéroaryle et un carboxyle ;
où e est un nombre entier compris entre 1 et 4 et où a, b, c, d, f et g sont, indépendamment les uns des autres, des nombres entiers compris entre 1 et 12 ;
où au moins l'un parmi Y, W et Z est choisi dans ledit deuxième groupe,
- avec une composition de carbonyle de formule générale (III) 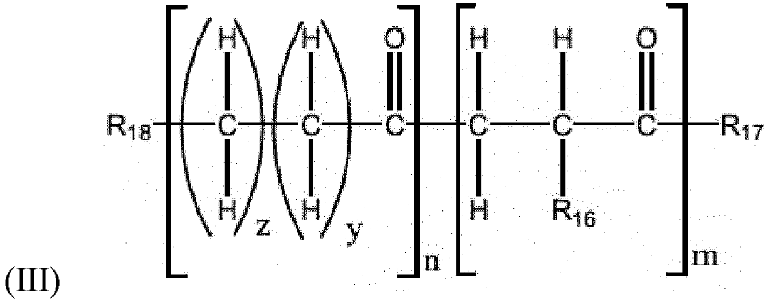
où n = 1-1000, m = 1-1000, y = 0-1, z = 0-1
où R₁₆ est choisi dans le groupe constitué par : un hydrogène et un méthyle ;
et R₁₇ et R₁₈ sont choisis dans le groupe constitué par : un hydrogène, un alkyle ou alcényle, un alcynyle en C₂ à C₃₀ linéaire ou ramifié, un aryle, un alkyl-aryle en C₂ à C₃₀, un alcényl-aryle en C₂ à C₃₀ linéaire ou ramifié, un alcynyl-aryle en C₂ à C₃₀ et un hétéroaryle ;
- et avec un oxyde-hydroxyde inorganique de sorte que la quantité en poids dudit oxyde-hydroxyde inorganique soit au moins dix fois supérieure à la somme de la quantité en poids de ladite composition d'amine et de ladite composition de carbonyle, et
b) apporter de l'énergie pour obtenir ledit adduit.

9. Procédé selon l'une des revendications 7 et 8, dans lequel ledit composé pyrrolique est oxydé avant d'être mélangé audit oxyde-hydroxyde inorganique.

10. Procédé selon la revendication 9, dans lequel ledit composé pyrrolique est oxydé par une composition donneuse d'oxygène choisie dans le groupe constitué par H₂O₂, MnO₂, KMnO₄, HNO₃, NaClO (hypochlorite de sodium), NaClO₂, H₂SO₄/HNO₃, NaIO₄, HIO₄, OsO₄, Pb(C₂H₃O₂)₄, HNO₃/HNO₂, CO₂, CO, le peroxyde de dicumyle, RuO₂, RuO₄, SeO₂, O₃,un periodinane, l'acide peracétique, l'acide iodosobenzoïque, le nicotinamide adénine dinucléotide (NAD+) et les enzymes laccase ou oxydase.

11. Procédé selon l'une quelconque des revendications 7 à 10, dans lequel ladite énergie est apportée en l'absence de solvants, de préférence dans lequel le mélange et l'apport d'énergie sont réalisés en l'absence de solvants.

12. Procédé selon l'une quelconque des revendications 7 à 10, dans lequel le mélange et l'apport d'énergie sont réalisés en présence d'au moins un solvant, de préférence choisi dans le groupe constitué par des alcools, des aldéhydes, des cétones, des esters, des éthers et de l'eau, plus préférablement un solvant à base d'eau.

13. Super-adduit, comprenant
a. un adduit selon l'une quelconque des revendications 1 à 6, comprenant ledit composé pyrrolique et ledit oxyde-hydroxyde inorganique, et
b. un allotrope de carbone hybridé en sp² ou ses dérivés.

14. Procédé de production d'un super-adduit selon la revendication 13, comprenant le fait de
a. fournir une troisième quantité en poids d'un adduit selon l'une quelconque des revendications 1 à 6, comprenant ledit composé pyrrolique et ledit oxyde-hydroxyde inorganique,
b. fournir une quatrième quantité en poids d'un allotrope de carbone hybridé en sp² ou de ses dérivés, et
c. mélanger ladite troisième quantité en poids dudit adduit avec ladite quatrième quantité en poids dudit allotrope de carbone hybridé en sp² ou de ses dérivés par apport d'énergie, afin d'obtenir ledit super-adduit.

15. Composition élastomère, comprenant un élastomère et une charge, dans laquelle ladite charge comprend un super-adduit selon la revendication 13.
